(19) Europäisches Patentamt
European Patent Office
Office européen des brevets

(11) **EP 4 714 979 A1**

(12) **EUROPEAN PATENT APPLICATION**
published in accordance with Art. 153(4) EPC

(43) Date of publication:
**25.03.2026 Bulletin 2026/13**

(21) Application number: **23936931.7**

(22) Date of filing: **15.05.2023**

(51) International Patent Classification (IPC):
*C07K 19/00* (2006.01)   *C07K 14/47* (2006.01)
*C12N 15/62* (2006.01)   *A61K 38/17* (2006.01)

(52) Cooperative Patent Classification (CPC):
**A61K 38/17; C07K 14/47; C07K 19/00; C12N 15/62**

(86) International application number:
**PCT/CN2023/094269**

(87) International publication number:
**WO 2024/234244 (21.11.2024 Gazette 2024/47)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Longbio Pharma (Suzhou) Co., Ltd.
Shanghai 201203 (CN)**

(72) Inventors:
• **MA, Haili
Shanghai 201203 (CN)**
• **LIU, Heng
Shanghai 201203 (CN)**

(74) Representative: **Bianchetti & Minoja with
Trevisan & Cuonzo IPS SRL
Via Plinio, 63
20129 Milano (IT)**

Remarks:
The complete document including Reference
Table(s) and the Sequence Listing(s) can be
downloaded from the EPO website

(54) **BIASED COMPLEMENT INHIBITORY HYBRID PROTEIN**

(57) The present invention relates to hybrid proteins comprising a CFH functional unit or a CR1 functional unit and a DAF functional unit, an encoding nucleic acid thereof, and a method and a use thereof for treating a complement system-associated disease or disorder.

## Description

## TECHNICAL FIELD

The present invention belongs to the fields of medical immunology and recombinant proteins, and specifically relates to a hybrid protein comprising functional units of different complement factors, an encoding nucleic acid thereof, and a method and a use thereof for treating a complement system-associated disease or disorder.

## BACKGROUND OF THE INVENTION

As part of the innate immune system, the complement system comprises more than 30 kinds of soluble protein molecules and more than 30 kinds of molecules such as intrinsic complement components, various regulatory factors, and complement receptors. It is well known that the complement system acts directly on pathogens to promote pathogen-specific adaptive immune responses; and participates in cell differentiation and polarization, tissue regeneration, lipid metabolism, clearance of immune complexes, and apoptosis, etc.

Depending on the initiating factors of activation, the complement activation occurs through three pathways: the classical pathway (CP), the alternative pathway (AP), and the lectin pathway (LP).

As the deficient or inappropriate complement activation regulation will lead to damage to host tissues, the complement system is tightly regulated by a series of proteins, wherein complement activation regulator (RCA) family proteins are primarily responsible for complement regulation; RCA proteins include membrane proteins such as decay-accelerating factor (DAF or CD55), membrane cofactor protein (MCP or CD46), complement receptor 1 (CR1 or CD35), complement receptor 2 (CR2 or CD21), CR1L, CRIg, Crry, CD59, as well as fluid-phase proteins such as Factor H ( factor H, FH or CFH) and C4b-binding protein (C4BP). The RCA protein structure consists of complement control protein repeat (CCP) modules, of which 2-4 consecutive modules contribute to regulatory functions known as decay-accelerating activity (DAA) and cofactor activity (CFA). RCA proteins act by targeting the C3/C5 convertase, the central enzyme of the complement pathway. RCA proteins bind to these convertases or their non-catalytic subunits to inactivate these enzymes by different mechanisms. The DAA activity was displayed as the irreversible dissociation of the invertase into subunits after binding to the RCA protein, thereby inactivating the invertase; whereas the CFA activity was displayed as the cleavage and inactivation of the invertase via the recruitment of serine protease Factor I (FI) after the binding RCA protein to the non-catalytic subunits (C3b/C4b), thereby preventing the formation of C3 convertase.

CFH is an opsonin and ligand for complement receptors 2 and 3, acting as a cofactor for Factor I in the catalyzed cleavage of C3b to iC3b, and inhibiting C3b amplification. It accelerates the irreversible dissociation of C3 convertase C3bBb in the alternative pathway, and may also compete with Factor B for C3B binding during the formation of proconvertase. Factor H is a soluble complement regulator essential for protecting surfaces, including extracellular matrix ECM. FH binds to the peptide hormone adrenomedullin and may prevent its degradation. The FH plays a role in managing cellular senescence, stress, or injury through interactions with C-reactive proteins, pentameric proteins, DNA, histones, annexin II, the malondialdehyde acetaldehyde adduct of proteins, and oxidized lipids. FHL1 also has cofactor activity for Factor I and C3bBb decay-accelerating activity (The Complement FactsBook. Edited by: Scott Barnum and Theresa Schein, Copyright© 2018 Elsevier Ltd. All rights reserved. https://doi.org/10.1016/C2015-0-06595-9, Chapter 30).

CR1 is a type-I transmembrane N-linked glycoprotein that is primarily expressed on blood cells and involved in innate immunity. The gene encoding CR1 is a member of the RCA cluster located on chromosome 1. The extracellular domain of CR1 contains binding sites of all primary complement fragments C3b, C4d, and C1q, as well as mannan-binding lectins and an Epstein-Barr virus (EBV). CR1 participates in the phagocytosis of complement opsonoid granules and immune complexes on circulating nucleated cells. The expression level in circulating red blood cells (RBCs) is genetically determined and varies among 90, 500, or 1200 CR1 copies/cell. In humans, the congenital CR1 defects are unknown. Acquired low expression levels of CR1 on circulating RBCs are seen during pregnancy and in a wide range of chronic and acute inflammatory diseases. Not CR1 but instead an alternative splicing variant of CR2 is expressed in mice (The Complement FactsBook. Edited by: Scott Barnum and Theresa Schein, Copyright © 2018 Elsevier Ltd. All rights reserved. https://doi.org/10.1016/C2015-0-06595-9, Chapter 28).

DAF intrinsically protects host cells from autologous complement attack by preventing the formation and accelerating the decay of classical and alternative C3 and C5 convertases, thereby inhibiting the cleavage of C3 and C5. When purified DAF is added to cells, it can be incorporated into the cell membrane, thus displaying functional activity. DAF has also been found to regulate T cell tolerance and thus negatively regulate several animal models of autoimmune diseases (The Complement FactsBook. Edited by: Scott Barnum and Theresa Schein, Copyright © 2018 Elsevier Ltd. All rights reserved. https://doi.org/10.1016/C2015-0-06595-9, Chapter 25).

Inhibiting complement activity using natural complement regulatory proteins holds therapeutic potential; however, complete or comprehensive inhibition of the complement has the risk of reducing immunity and increasing infection.

For example, treatment with the C5 antibody drug SOLIRIS requires prior meningococcal vaccination, and treatment with the C3 inhibitor EMPAVELI increases the risk of infection with *meningococcus, Streptococcus pneumoniae, Neisseria meningitidis,* and *Haemophilus* influenzae type B. Because different RCAs or RCA domains exhibit biased complement inhibitory activities, for example, FH and FHL1 are more biased towards inhibiting AP activity, DAF has inhibitory activity against both AP and CP, and different fragment regions of CR1 also have different activities, therefore, hybridizing functional units of different RCAs could potentially yield potent and precise complement inhibitory proteins with biased activities, thereby providing better options for the treatment of complement-related diseases.

**SUMMARY OF THE INVENTION**

The present invention provides a hybrid protein containing functional units of different complement factors. This hybrid protein exhibits biased inhibition of complements, with reduced inhibition activity toward Classical Pathway (CP) and enhanced inhibition activity toward Alternative Pathway (AP), thus the immune function of the CP pathway can be preserved or partially preserved while treating a disease caused by AP activity (such as PNH). The present invention thus also provides a method for precisely treating dysregulation of complement AP activity.

Specifically, the present invention provides a hybrid protein of CFH and DAF, wherein CCP1-2/SCR1-2 of CFH is hybridized with CCP of DAF (e.g., CCP4/SCR4 of DAF) to form the hybrid protein.

The present invention also provides a hybrid protein of CR1 and DAF, wherein CCP15/SCR15 of CR1 is hybridized with CCP of DAF (e.g., CCP3-4/SCR3-4 of DAF) to form the hybrid protein.

In some embodiments, the hybrid protein of the present invention is effective in inhibiting hemolysis mediated by the human complement pathway. In some particular embodiments, the hybrid protein of the present invention is effective in inhibiting hemolysis mediated by the human complement alternative pathway.

In some embodiments, the hybrid protein of the present invention having a smaller molecular weight can be administered at the same mass concentration with higher molarity doses, and thus have the potential of better therapeutic efficacy or longer dosing intervals, e.g. in ophthalmic, brain medications, as compared to other typically used complement regulatory proteins with larger molecular weight. In some embodiments, the hybrid protein of the present invention has more significant tissue penetration properties, such as tumor tissue, blood-brain barrier, and the like.

In some embodiments, the present invention relates to the following specific items:

1. A hybrid protein, which comprises or consists of

(i) CCP1-2 of human Complement Factor H (CFH),
(ii) CCP4 of human decay accelerating factor (DAF), and

optionally a signal peptide and/or a tag.
2. The hybrid protein of embodiment 1, wherein the CCP1 and CCP2 of CFH are directly linked together to form CCP1-2.
3. The hybrid protein of embodiment 1 or 2, wherein the CCP1 of human CFH comprises or consists of the amino acid sequence at positions 19-82 of the human CFH protein; and/or the CCP2 of human CFH comprises or consists of the amino acid sequence at positions 83-143 of the human CFH protein;

wherein optionally, the CCP1 comprises a V62I mutation,
wherein optionally, the CCP2 comprises one, two, or three of E116T, Q119K, and V143E mutations,
wherein the amino acid positions are numbered corresponding to the amino acid positions as set forth in SEQ ID NO: 3.

4. The hybrid protein of embodiment 1 or 2, wherein the CCP1-2 of human CFH comprises or consists of the amino acid sequence at positions 19-143 of the human CFH protein,

wherein optionally, the CCP1-2 comprises one, two, three, or four of V62I, E116T, Q119K, and V143E mutations,
wherein the amino acid positions are numbered corresponding to the amino acid positions as set forth in SEQ ID NO: 3.

5. The hybrid protein of any one of embodiments 1-4, wherein the CCP4 of human DAF comprises or consists of the amino acid sequence at positions 223-285 of the human DAF protein, wherein the amino acid positions are numbered corresponding to the amino acid positions as set forth in SEQ ID NO: 1.
6. The hybrid protein of any one of embodiments 1-4, wherein the CCP4 of human DAF comprises or consists of the amino acid sequence at positions 223-287 of the human DAF protein, wherein the amino acid positions are numbered corresponding to the amino acid positions as set forth in SEQ ID NO: 1.

7. The hybrid protein of embodiment 2, wherein

(1) the CCP1 of human CFH comprises or consists of the amino acid sequences at positions 19-82 of the human CFH protein, and the CCP2 of human CFH comprises or consists of the amino acid sequence 83-143 of the human CFH protein, and the CCP4 of human DAF comprises or consists of the amino acid sequence 223-285 of the human DAF protein;

(2) the CCP1 of human CFH comprises or consists of the amino acid sequences at positions 19-82 of the human CFH protein, and the CCP2 of human CFH comprises or consists of the amino acid sequence 83-143 of the human CFH protein, and the CCP4 of human DAF comprises or consists of the amino acid sequence 223-287 of the human DAF protein;

(3) the CCP1-2 of human CFH comprises or consists of the amino acid sequence at positions 19-143 of the human CFH protein, and the CCP4 of human DAF comprises or consists of the amino acid sequence at positions 223-285 of the human DAF protein; or

(4) the CCP1-2 of human CFH comprises or consists of the amino acid sequence at positions 19-143 of the human CFH protein, and the CCP4 of human DAF comprises or consists of the amino acid sequence at positions 223-287 of the human DAF protein;

wherein the amino acid positions of the human CFH protein are numbered corresponding to the amino acid positions as set forth in SEQ ID NO: 3 and the amino acid positions of the human DAF protein are numbered corresponding to the amino acid positions as set forth in SEQ ID NO: 1.

8. The hybrid protein of embodiment 7, wherein the CCP1 of human CFH comprises a V62I mutation, the CCP2 of human CFH comprises one, two, or three of E116T, Q119K, and V143E mutations, and/or the CCP1-2 of human CFH comprises one, two, three, or four of V62I, E116T, Q119K, and V143E mutations.

9. The hybrid protein of any one of embodiments 1-8, wherein the human CFH protein is a native human CFH protein or comprises

(i) the amino acid sequence as set forth in SEQ ID NO: 3 or 5;
(ii) the amino acid sequence encoded by the nucleic acid sequence as set forth in SEQ ID NO: 4 or 6;
(iii) the amino acid sequence having at least 95%, 96%, 97%, 98%, or 99% identity to the amino acid sequence as set forth in (i) or (ii); or

consists of the amino acid sequence as set forth in any one of (i) to (iii).

10. The hybrid protein of any one of embodiments 1-9, wherein the human DAF protein is a native human DAF protein or comprises

(i) the amino acid sequence as set forth in SEQ ID NO: 1;
(ii) the amino acid sequence encoded by the nucleic acid sequence as set forth in SEQ ID NO: 2;
(iii) the amino acid sequence having at least 95%, 96%, 97%, 98%, or 99% identity to the amino acid sequence as set forth in (i) or (ii); or

consists of the amino acid sequence as set forth in any one of (i) to (iii).

11. The hybrid protein of any one of embodiments 1-10, wherein

the CCP1 of human CFH comprises or consists of the amino acid sequence as set forth in SEQ ID NO: 14 or 15, or an amino acid sequence having at least 95%, 96%, 97%, 98%, or 99% identity to the amino acid sequence as set forth in SEQ ID NO: 14 or 15;

the CCP2 of human CFH comprises or consists of the amino acid sequence as set forth in SEQ ID NO: 16 or 17, or an amino acid sequence having at least 95%, 96%, 97%, 98%, or 99% identity to the amino acid sequence as set forth in SEQ ID NO: 16 or SEQ ID NO: 17;

the CCP1-2 of human CFH comprises or consists of the amino acid sequence as set forth in SEQ ID NO: 31, 32, 33, or 34, or an amino acid sequence having at least 95%, 96%, 97%, 98%, or 99% identity to the amino acid sequence as set forth in SEQ ID NO: 31, 32, 33, or 34; and/or

the CCP4 of human DAF comprises or consists of the amino acid sequence as set forth in SEQ ID NO: 10 or SEQ ID NO: 11, or an amino acid sequence having at least 95%, 96%, 97%, 98%, or 99% identity to the amino acid sequence as set forth in SEQ ID NO: 10 or SEQ ID NO: 11.

12. The hybrid protein of any one of embodiments 1-11, wherein the tag is, for example, a purification tag such as a hexahistidine tag or a biotin tag, comprising, e.g., the amino acid sequence as set forth in SEQ ID NO: 19.

13. The hybrid protein of any one of embodiments 1-12, wherein the signal peptide is secretory, comprising, e.g., the amino acid sequence as set forth in SEQ ID NO: 20.

14. The hybrid protein of any one of embodiments 1-13, wherein the hybrid protein comprises or consists of the amino acid sequence of any one of SEQ ID NOs: 21-28, or an amino acid sequence having at least 95%, 96%, 97%, 98%, or 99% identity to the amino acid sequence.

15. A hybrid protein, which comprises or consists of

    (i) CCP15 or CCP8 of human complement receptor 1 (CR1),
    (ii) CCP3-4 of human decay accelerator factor (DAF), and

optionally a signal peptide and/or a tag.

16. The hybrid protein of embodiment 15, wherein the CCP3 and CCP4 of DAF are directly linked together to form CCP3-4.

17. The hybrid protein of embodiment 15 or 16, wherein CCP15 of human CR1 comprises the amino acid sequence at positions 941-1001 of the human CR1 protein, and CCP8 comprises the amino acid sequence at positions 491-551 of the human CR1 protein, wherein the amino acid positions are numbered corresponding to the amino acid positions as set forth in SEQ ID NO: 7.

18. The hybrid protein of any one of embodiments 15-17, wherein CCP3 of human DAF comprises or consists of the amino acid sequence at positions 161-222 of the human DAF protein, and/or CCP4 of human DAF comprises or consists of the amino acid sequence at positions 223-285 of the human DAF protein, wherein the amino acid positions are numbered corresponding to the amino acid positions as set forth in SEQ ID NO:1.

19. The hybrid protein of any one of embodiments 15-17, wherein CCP3 of human DAF comprises or consists of the amino acid sequence at positions 161-222 of the human DAF protein, and/or CCP4 of human DAF comprises or consists of the amino acid sequence at positions 223-287 of the human DAF protein, wherein the amino acid positions are numbered corresponding to the amino acid positions as set forth in SEQ ID NO:1.

20. The hybrid protein of embodiment 16 or 17, wherein the CCP3-4 of human DAF comprises or consists of the amino acid sequence at positions 161-285 of the human DAF protein, wherein the amino acid positions are numbered corresponding to the amino acid positions as set forth in SEQ ID NO: 1.

21. The hybrid protein of embodiment 16 or 17, wherein the CCP3-4 of human DAF comprises or consists of the amino acid sequence at positions 161-287 of the human DAF protein, wherein the amino acid positions are numbered corresponding to the amino acid positions as set forth in SEQ ID NO: 1.

22. The hybrid protein of embodiment 16, wherein

    (1) the CCP15 of human CR1 comprises or consists of the amino acid sequence at positions 941-1001 of the human CR1 protein, and the CCP3 of human DAF comprises or consists of the amino acid sequence at positions 161-222 of the human DAF protein, and the CCP4 of human DAF comprises or consists of the amino acid sequence at positions 223-285 of the human DAF protein; or
    (2) the CCP15 of human CR1 comprises or consists of the amino acid sequence at positions 941-1001 of the human CR1 protein, and the CCP3 of human DAF comprises or consists of the amino acid sequence at positions 161-222 of the human DAF protein, and the CCP4 of human DAF comprises or consists of the amino acid sequence at positions 223-287 of the human DAF protein; or
    (3) the CCP15 of human CR1 comprises or consists of the amino acid sequence at positions 941-1001 of the human CR1 protein, and the CCP3-4 of human DAF comprises or consists of the amino acid sequence at positions 161-285 of the human DAF protein;
    (4) the CCP15 of human CR1 comprises or consists of the amino acid sequence at positions 941-1001 of the human CR1 protein, and the CCP3-4 of human DAF comprises or consists of the amino acid sequence at positions 161-287 of the human DAF protein;

    wherein the amino acid positions of the human CR1 protein are numbered corresponding to the amino acid positions as set forth in SEQ ID NO:7,
    and the amino acid positions of the human DAF protein are numbered corresponding to the amino acid positions as set forth in SEQ ID NO:1.

23. A hybrid protein of any one of embodiments 16-22, wherein the human CR1 protein is a native human CR1 protein or comprises:

    (i) the amino acid sequence as set forth in SEQ ID NO: 7;

(ii) the amino acid sequence encoded by the nucleic acid sequence as set forth in SEQ ID NO: 8;
(iii) the amino acid sequence having at least 95%, 96%, 97%, 98%, or 99% identity to the amino acid sequence as set forth in (i) or (ii); or

consists of the amino acid sequence as set forth in any one of (i) to (iii).

24. The hybrid protein of any one of embodiments 15-23, wherein the human DAF protein is a native human DAF protein or comprises

(i) the amino acid sequence as set forth in SEQ ID NO: 1;
(ii) the amino acid sequence encoded by the nucleic acid sequence as set forth in SEQ ID NO: 2;
(iii) the amino acid sequence having at least 95%, 96%, 97%, 98%, or 99% identity to the amino acid sequence as set forth in (i) or (ii); or

consists of the amino acid sequence as set forth in any one of (i) to (iii).

25. The hybrid protein of any one of embodiments 18-24, wherein

the CCP15 or CCP8 of human CR1 comprises or consists of the amino acid sequence as set forth in SEQ ID NO: 18, or an amino acid sequence having at least 95%, 96%, 97%, 98%, or 99% identity to the amino acid sequence as set forth in SEQ ID NO: 18;
the CCP3 of human DAF comprises or consists of the amino acid sequence as set forth in SEQ ID NO: 9, or an amino acid sequence having at least 95%, 96%, 97%, 98%, or 99% identity to the amino acid sequence as set forth in SEQ ID NO: 9;
the CCP4 of human DAF comprises or consists of the amino acid sequence as set forth in SEQ ID NO: 10 or SEQ ID NO: 11, or an amino acid sequence having at least 95%, 96%, 97%, 98%, or 99% identity to the amino acid sequence as set forth in SEQ ID NO: 10 or SEQ ID NO: 11;
the CCP3-4 of human DAF comprises or consists of the amino acid sequence as set forth in SEQ ID NO:12 or SEQ ID NO:13, or an amino acid sequence having at least 95%, 96%, 97%, 98%, or 99% identity to the amino acid sequence as set forth in SEQ ID NO:12 or SEQ ID NO:13.

26. The hybrid protein of any one of embodiments 16-25, wherein the tag is, for example, a purification tag such as a hexahistidine tag or a biotin tag, comprising, e.g., the amino acid sequence as set forth in SEQ ID NO: 19.

27. The hybrid protein of any one of embodiments 16-26, wherein the signal peptide is secretory, comprising, e.g., the amino acid sequence as set forth in SEQ ID NO: 20.

28. The hybrid protein of any one of embodiments 16-27, wherein the hybrid protein comprises or consists of the amino acid sequence as set forth in SEQ ID NO: 29 or 30, or an amino acid sequence having at least 95%, 96%, 97%, 98%, or 99% identity to the amino acid sequence.

29. A nucleic acid molecule encoding the hybrid protein of any one of embodiments 1-28.

30. An expression vector comprising the nucleic acid molecule of embodiment 29, preferably the expression vector is pCDNA3.1.

31. A host cell comprising the nucleic acid molecule of embodiment 29 or the expression vector of embodiment 30, preferably the host cell is prokaryotic or eukaryotic, such as a 293 cell, e.g., an Expi293 cell.

32. A method of preparing the hybrid protein of any one of embodiments 1-28, the method comprising culturing the host cell of embodiment 31 under conditions suitable for expression of the hybrid protein, and optionally further comprising isolating the protein from the host cell or the host cell culture medium, and/or purifying the protein.

33. A pharmaceutical composition or medicament or formulation comprising the hybrid protein according to any one of embodiments 1-28 or a nucleic acid molecule encoding the same, or the nucleic acid molecule of embodiment 29, and optionally a pharmaceutically acceptable excipient.

34. A pharmaceutical combination product comprising

the hybrid protein according to any one of embodiments 1-28 or a nucleic acid molecule encoding the same, or the nucleic acid molecule of embodiment 29; and
the additional therapeutic agents.

35. A method of preventing or treating a complement system-associated disease or disorder in a subject, comprising administering to the subject an effective amount of the hybrid protein of any one of embodiments 1-28 or a nucleic acid molecule encoding the same; or the nucleic acid molecule of embodiment 29; or the pharmaceutical composition or formulation of embodiment 33; or the pharmaceutical combination product of embodiment 34.

36. The method of embodiment 35, wherein the disease or disorder is due to an abnormal activation of the complement system or a dysregulation of the complement system.

37. The method of embodiment 36, wherein the abnormal activation of the complement system or the dysregulation of the complement system is due to for example, a microbial infection or an increase in autoimmune antibodies, or a decrease, deletion, incapacitation, or interference or blockade of a function of complement regulatory proteins.

38. The method of embodiment 37, wherein the complement system-associated disease or disorder is selected from diseases caused by activation of the alternative pathway, such as a disease requiring inhibition of the alternative pathway of the complement immunity and optionally not inhibition of classical pathway of the complement immunity, such as paroxysmal nocturnal hemoglobinuria (PNH).

## BRIEF DESCRIPTION OF THE DRAWINGS

Figure 1 shows the crystal structures of the CFH protein CCP1-4 domain portions and the DAF protein having CCP1-4 domains, and the structural model of the biased F12D4 hybrid protein formed by CFH CCP1-2 and DAF CCP4.

Figure 2 shows the reduced and non-reduced SDS-PAGE electrophoresis pattern of various recombinant complement regulatory proteins constructed in the present invention after expressed in Expi293 cells. The legend indicates the types of proteins in each lane.

Figure 3 shows the purity results of the biased hybrid protein F12D4 as determined by HPLC-SEC.

Figure 4 shows the purity results of the biased hybrid protein CR15D34 constructed by CR1 CCP 15and DAF CCP3-4, as determined by HPLC-SEC.

Figure 5 shows the CP inhibitory activity of each complement regulatory protein.

Figure 6 shows the AP inhibitory activity of each complement regulatory protein.

## DETAILED DESCRIPTION OF THE INVENTION

### I. Definitions

It should to be understood that this invention is not limited to the specific methodologies, protocols, and reagents described herein, as these may vary. It should also be understood that the terminology used herein is for describing particular embodiments only, and is not intended to limit the scope of the present invention which will be limited only by the appended claims. Unless otherwise specified, all technical and scientific terms used herein have the same meanings as those generally understood by a person of ordinary skill in the art to which the present invention belongs.

### ➢ Decay-accelerating Factor (DAF)

Decay-accelerating Factor (DAF, CD55) is a membrane-associated regulatory protein that protects autologous cells from activation of autologous complement on their surfaces. DAF acts by rapidly dissociating C3 and C5 convertases (central enzymes of the cascade). DAF has the most potent attenuation-accelerating activity of proteins associated with complement regulation and acts on classical pathway enzymes (C4b2a and C4b2a3b) and alternative pathway enzymes (C3bBb and C3BbC3b). However, DAF has no cofactor function.

Structural analysis of the DAF shows that, starting from its N-terminus, it consists of a unit of 4-60 amino acids in length, followed by a fragment (STP) rich in highly O-glycosylated serine (S) and threonine (T), followed by a post-translationally added glycosylphosphatidylinositol (GPI) anchor.

In some embodiments, the DAF is a human DAF having the following accession numbers: Genbank accession numbers M31516, M15799, M64653, S72858, or M643567.

In some embodiments, the DAF is human DAF. In some embodiments, the human DAF is native human DAF. In some embodiments, the amino acid sequence of native human DAF is set forth in SEQ ID NO: 1, with the various modules shown in Table 1 below. Four 60 amino acid long repeating units termed complement control protein repeats (CCP) or Short Consensus Repeats (SCR). CCP1 includes amino acids 35-96; CCP2 includes amino acids 96-160; CCP3 includes amino acids 161-222 and CCP4 includes amino acids 223-285. They provide all the regulatory activities of DAF. The highly O-glycosylated region serves as a cushion that positions the CCP at an appropriate distance suspended above the surface membrane. The GPI anchor allows DAF to move freely on the plasma membrane, making it possible to inactivate invertase complexes anywhere they are assembled. In this context, reference to the amino acid positions of the modules of DAF is made to the amino acid position numbering corresponding to that as set forth in SEQ ID NO: 1.

In some embodiments, the amino acid positions corresponding to each module of the DAF are shown in Table 1 below. In some embodiments, where otherwise defined in the context, the amino acid positions corresponding to each module of the DAF are adjusted based on the positions noted in Table 1 (e.g., by truncation or elongation of 1-2 amino acid residues in the N-and/or C-terminal direction)

Table 1: Protein modules of DAF (divided by UniProt way)

| The amino acid position of SEQ ID NO:1 | Module |
|---|---|
| 1 -34 | Lead sequence |
| 35 -96 | CCP1 |
| 96 -160 | CCP2 |
| 161 -222 | CCP3 |
| 223 -285 | CCP4 |
| 287 -356 | STP |

In some embodiments, the nucleotide sequence of the cDNA encoding DAF is set forth in SEQ. ID NO: 2.

➢ **Factor H (CFH or FH)**

"Complement Factor H", "Factor H", "FH", "CFH protein" or "CFH" are used interchangeably and refer to a protein of approximately 150 kDa that is a member of the regulators of the complement activation family and is a complement control protein. CFH is a large soluble glycoprotein that circulates in human plasma and serves to regulate the alternative pathway of the complement system, ensuring that the complement system is directed towards pathogens or other dangerous substances and does not damage host tissues.

Factor H is predominantly monomeric but can be weakly self-associated (KD = 28 $\mu$M) and may be oligomeric in the presence of glycosaminoglycans or high concentrations of metal ions. CFH consists of 20 homologous units called complement control protein repeats (CCPs) (SCR or sushi domains), some of which function for cell attachment, while other repeats function to eliminate C3b from the cell surface. Each of the 20 SCRs is about 60 amino acids in length, arranged head to tail, comprising 4 cysteine residues, forming 2 disulfide bonds for each module. SCR 19 and 20 are involved in C3b binding.

The splice variant FHL-1 consists of the first 7 CCPs, followed by the C-terminal sequence Ser-Pro-Leu-Thr. Each CCP comprises approximately 60 residues, including four invariant cysteines, forming Cys$^I$-Cys$^{III}$, Cys$^{II}$-Cys$^{IV}$ disulfides. Adjacent modules are connected by sequences of three to eight residues.

The images from negative staining electron microscopy show that FH molecules adopt a variety of conformations, but are mainly folded in half; analytical ultracentrifugation, small angle X-ray scattering (deposition of the module in PDB, e.g., 3 GAV), and chemical cross-linking also indicated that the module chain itself was bent. The following high resolution structure of several FH fragments was determined, alone or in complex with other molecules (PDB identifier is provided in parenthesis below): CCP 1-2 (2RLP), 2-3 (2RLQ), 1-4 (2WII), 5, 6-7 (e.g., 2W80, 2 YBY), 7 (2JGW and 2JGX), 6-8 (2 UWN and 2V8E), 9 (4K12), 10-11 (4B2R), 11-12 (4B2S), 12-13 (2KMS), 15 (1HFI), 16 (1HCC), 15-16 (1HFH), 18-20 (3SWO), 19-20 (e.g., 2BZM, 2G7I and 4ONT). Each CCP resembles a prolate spheroid having a major axis of about 4 nm and a minor axis of about 2 nm and comprises beta strands in anti-parallel patches that are approximately aligned with the major axis, with the N- and C-termini located at either end of its long axis, facilitating an end-to-end arrangement of tandem CCPs with variable inter-module contact, tilt, and twists.

In some embodiments, the CFH is a human CFH, e.g., a native human CFH. In some embodiments, the amino acid sequence of CFH has the following accession numbers: HGNC: HGNC: 4883, Ensembl: ENSG00000000971, HPRD: 00601, MIM:134370 Vega: OTTHUMG00000035607.

In some embodiments, the amino acid sequence of CFH is set forth in SEQ ID NO: 3. In some embodiments, the amino acid positions corresponding to each module of the CFH are shown in Table 2 below. In some embodiments, where otherwise defined in the context, the amino acid positions corresponding to each module of the CFH are adjusted based on the positions as listed in Table 2 (e.g., by truncation or elongation of 1-2 amino acid residues in the N-and/or C-terminal direction)

Table 2: Protein modules of CFH (divided by UniProt way)

| The amino acid position of SEQ ID NO:3 | Module |
|---|---|
| 1-18 | Lead peptide |
| 19 -82 | CCP1 |
| 83 -143 | CCP2 |

(continued)

| The amino acid position of SEQ ID NO:3 | Module |
|---|---|
| 144 -207 | CCP3 |
| 208 -264 | CCP4 |
| 265 -322 | CCP5 |
| 324 -386 | CCP6 |
| 387 -444 | CCP7 |
| 446 -507 | CCP8 |
| 515-566 | CCP9 |
| 567 -625 | CCP10 |
| 628 -686 | CCP11 |
| 689 -746 | CCP12 |
| 751 -805 | CCP13 |
| 809 -866 | CCP14 |
| 868 -928 | CCP15 |
| 929 -986 | CCP16 |
| 987 -1045 | CCP17 |
| 1046 -1104 | CCP18 |
| 1107 -1165 | CCP19 |
| 1170 -1230 | CCP20 |

In some embodiments, the nucleotide sequence of the cDNA encoding CFH is set forth in SEQ. ID NO: 4.

In some embodiments, the protein sequence encoding the splice variant FHL-1 of CFH is set forth in SEQ ID NO: 5. In some embodiments, the nucleotide sequence of the cDNA encoding the splice variant FHL-1 of CFH is set forth in SEQ ID NO: 6.

In this context, reference to the amino acid positions of the modules of CFH is made to the amino acid position numbering corresponding to that as set forth in SEQ ID NO: 3.

### ➢ Complement receptor 1 (CR1)

Terms "complement receptor type 1", "CR1," or "CD35" are used interchangeably and refer to the complement C3b/C4d receptor membrane protein of the complement activating receptor family, which has the most common splice variant, CR1 isoform F precursor, consisting of 2039 amino acids with a molecular weight of approximately 220 kDa. CR1 is mainly expressed in erythrocytes, monocytes/macrophages, neutrophils, and B cells, and is also present in some T lymphocytes, mast cells, and glomerular podocytes. As the name suggests, the most important ligands for CR1 are C3b/C4d, mediating complement opsonization and promoting the clearance of immune complexes. It has been shown that CR1 can act as a negative regulator of the complement cascade, mediating immune adhesion and phagocytosis, and inhibiting both classical and alternative pathways.

Taking the most common splice variant as an example, the full-length CR1 protein comprises a signal peptide of 42 amino acid residues, an extracellular domain of 1930 amino acid residues, a transmembrane domain of 25 amino acid residues, and a C-terminal cytoplasmic domain of 43 amino acid residues. The extracellular domain of CR1 has 25 potential N-glycosylation signal sequences and comprises 30 CCP domains, with each approximately 60-70 amino acids in length. The sequence homology among these CCP domains is 60-99%.

In some embodiments, CR1 is human CR1, such as the native human CR1. In some embodiments, the amino acid sequence of CFH has the accession numbers as follows: Uniprot accession number P17927, Genbank accession numbers NP_000564.2, NP_000642.3, and NP_001368780.1.

In some embodiments, the amino acid sequence of CR1 is set forth in SEQ ID NO: 7. In some embodiments, the amino acid positions corresponding to each module of the CR1 are shown in Table 3 below. In some embodiments, where otherwise defined in the context, the amino acid positions corresponding to each module of the CR1 are adjusted based on the positions noted in Table 3 (e.g., by truncation or elongation of 1-2 amino acid residues in the N-and/or C-terminal direction)

Table 3: Protein modules of CR1 (divided by UniProt way)

| The amino acid position of SEQ ID NO:7 | Module |
|---|---|
| 1-41 | Lead peptide |
| 42 -101 | CCP1 |
| 102 -163 | CCP2 |
| 164 -234 | CCP3 |
| 236 -295 | CCP4 |
| 295 -355 | CCP5 |
| 356 -418 | CCP6 |
| 419 -489 | CCP7 |
| 491 -551 | CCP8 |
| 552 -613 | CCP9 |
| 614 -684 | CCP10 |
| 686 -745 | CCP11 |
| 745 -805 | CCP12 |
| 806 -868 | CCP13 |
| 869 -939 | CCP14 |
| 941 -1001 | CCP15 |
| 1002 -1063 | CCP16 |
| 1064 -1134 | CCP17 |
| 1136 -1195 | CCP18 |
| 1195 -1255 | CCP19 |
| 1256 -1318 | CCP20 |
| 1319 -1389 | CCP21 |
| 1394 -1454 | CCP22 |
| 1455 -1516 | CCP23 |
| 1517 -1587 | CCP24 |
| 1589 -1648 | CCP25 |
| 1648 -1708 | CCP26 |
| 1709 -1771 | CCP27 |
| 1772 -1842 | CCP28 |
| 1846 -1906 | CCP29 |
| 1907 -1967 | CCP30 |
| 2017 -2039 | C-terminal cytoplasmic domain |

In some embodiments, the nucleotide sequence of the cDNA encoding CR1 is set forth in SEQ. ID NO: 8.
In this context, reference to the amino acid positions of the modules of CR1 is made to the amino acid position numbering corresponding to that as set forth in SEQ ID NO: 7.

➢ **Other definitions**

For the purpose of interpreting this specification, the following definitions will be used and, wherever appropriate, terms used in the singular may also include the plural and vice versa. It is to be understood that the terminology used herein is for the purpose of describing particular embodiments only and is not intended to be limiting.

The term "about" when used in conjunction with a numerical value is intended to encompass a range of numerical values having a lower limit that is 5% less than the specified numerical value and an upper limit that is 5% greater than the specified numerical value.

As used herein, the term "and/or" means any one of the options or two or more or all of the options.

As used herein, the term "comprises" or "includes" means the inclusion of the stated element, integer, or step, but not the exclusion of any other element, integer, or step. When the term "comprises" or "includes" is used herein, it also encompasses a combination of the stated elements, integers, or steps unless otherwise indicated. For example, reference to a protein "comprising" a specific sequence is intended to also encompass the protein consisting of that specific sequence.

The terms "protein" and "polypeptide" are used interchangeably throughout this application, referring to polymeric sequences comprising amino acid residues. Both single-letter and three-letter codes for amino acids, as defined by the Joint Commission on Biochemical Nomenclature (JCBN) of IUPAC-IUB, are used throughout this application, unless otherwise specified. The single letter "X" refers to any of the twenty amino acids. It should also be understood that, due to the degeneracy of the genetic code, a polypeptide may be encoded by more than one nucleotide sequence.

As used herein, the terms "hybrid protein" and "chimeric polypeptide" are used interchangeably and refer to polypeptide formed by the fusion of at least two heterologous polypeptide sequences, optionally through a linker. Hybrid proteins may be produced by recombinant expression.

"Percent identity (%)" of an amino acid sequence refers to the percentage of amino acid residues in a candidate sequence that are identical to the amino acid residues of the specific amino acid sequence shown in this specification, after aligning the candidate sequence with the specific amino acid sequence shown in this specification and introducing gaps, if necessary, to achieve the maximum percent sequence identity, and without taking into account any conservative substitutions included as part of sequence identity. In some embodiments, the present invention contemplates variants of a protein or polypeptide of the present invention that have a substantial degree of identity, e.g., at least 80%, 85%, 90%, 95%, 97%, 98%, or 99% or more identity, relative to the polypeptide or protein specifically disclosed herein. The variants may comprise conservative alterations. The variant may comprise conservative alterations.

For polypeptide sequences, "conservative alterations" include substitutions, deletions, or additions to the polypeptide sequence that do not substantially change the desired functional activity of the polypeptide sequence. For example, conservative substitutions often result in the replacement of an amino acid with a chemically similar amino acid. Conservative substitution tables providing functionally similar amino acids are well known in the art. 8 groups of amino acids conservatively substituted for each other are listed as follows: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M) ), Valine (V); 6) Phenylalanine (F), Tyrosine (Y), Tryptophan (W); 7) Serine (S), Threonine (T); and 8) Cysteine (C), Methionine (M). In some embodiments, the term "conservative sequence alteration" is used to refer to amino acid modifications that do not significantly affect or alter the activity of the parent hybrid protein. For example, conservatively modified variants retain at least 80%, 85%, 90%, 95%, 98%, 99%, or more, e.g., 100-110% or more activity relative to the parent polypeptide or hybrid protein. Herein, as used by those skilled in the art, mutations/substitutions in an amino acid sequence may be named as follows: a single-letter code for the parent amino acid, followed by the position number, and then a single-letter code for the variant amino acid. For example, mutating valine (V) at position 62 to isoleucine (I) is represented as "V62I". Unless otherwise explicitly defined or stated, the position number generally refers to the position number of the natural full-length polypeptide/protein (even when referring to positions in fragments/truncated forms), such as the position number of the most common splice variants.

The term "host cell" refers to a cell into which an exogenous polynucleotide has been introduced, including the progeny of such cells. Host cells include "transformants" and "transformed cells," which include primary transformed cells and progeny derived therefrom. Host cells are any type of cell system that can be used to produce hybrid proteins of the present invention, including eukaryotic cells, such as mammalian cells, insect cells, yeast cells; and prokaryotic cells, such as E. coli cells. Host cells include cultured cells, as well as cells within transgenic animals, transgenic plants, or cultured plant tissue or animal tissue.

The term "expression vector" refers to a vector comprising a recombinant polynucleotide comprising expression control sequences operably linked to the nucleotide sequence to be expressed. The expression vector comprises sufficient cis-acting elements for expression; other elements for expression can be provided by the host cell or in an in vitro expression system. Expression vectors include all those known in the art, including cosmids, plasmids (e.g., naked or included in liposomes), and viruses (e.g., lentiviruses, retroviruses, adenoviruses, viruses, and adeno-associated viruses) into which recombinant polynucleotides are incorporated.

The terms "classical pathway" or "CP" refers to one of the activation pathways of the complement system. As well known in the art, in general, the complement system of higher mammals (e.g., Homo sapiens) can be activated via three pathways: the classical pathway, the alternative pathway (or bypass pathway), and the lectin pathway. The classical pathway is activated by the binding of the corresponding activating factor (mainly the antigen-antibody complex) to C1. C1q binds to a single IgM molecule or two contiguous IgG molecules, for example, an IgG "duplicate" binds to the antigen and reacts with C1q to activate C1, which in turn activates C1r and C1s. This further activates downstream complement molecules in a cascade of reactions of C1, 4, 2, 3, 5, 6, 7, 8, and 9 in the classical complement activation pathway. In this cascade, C3 is cleaved by the classical pathway C3 convertase C4b2a to obtain C3b, which then reacts with C4b2a to form the classical pathway C5 convertase: C4b2a3b, which catalyzes the cleavage of C5 to obtain C5b. C5b combines with C6 and C7 to form the C5b67 complex, which ultimately links with C8 and C9 molecules to form the C5b6789 complex, i.e., the membrane-attack complex. This complex can create a pore of about 10 nm in the cell membrane, causing the target cell to swell and rupture due to insufficient osmotic pressure. The cleavage of C3 catalyzed by C3 convertase to C3b is a common downstream route of the complement activation pathway.

The terms "alternative pathway" or "bypass pathway" or "AP" refers to one of the activation pathways of the complement system. Unlike the classical pathway, which often requires a specific immune response (antigen-antibody complex), in the alternative pathway, under the activation by substances such as the bacterial cell wall, polysaccharides, peptidoglycans, teichoic acid, and aggregated IgA and IgG4, C3b formed by the spontaneous cleavage of C3 is bound by factors B, D, and P to form C3 convertase, which exists stably and gradually accumulates to a certain level, triggering downstream cascade reactions.

The terms "individual" or "subject" are used interchangeably herein to refer to mammals. Mammals include, but are not limited to, domesticated animals (e.g., cows, sheep, cats, dogs, and horses), primates (e.g., humans and non-human primates such as monkeys), rabbits, and rodents (e.g., mice and rats). In particular, the individual is a human.

The term "treating" includes administering the composition or hybrid polypeptide to prevent or delay the onset of the symptoms, complications, or biochemical indicators of a disease, alleviate the symptoms, or arrest or inhibit the further development of the disease, condition, or disorder. The term "preventing" includes the inhibition of the occurrence or development of a disease or disorder or the symptoms of a particular disease or disorder.

The term "pharmaceutically acceptable auxiliary material" refers to diluents, adjuvants (e.g., Freund's adjuvants (complete and incomplete)), excipients, carriers, or stabilizers, etc., with which the active substances is administered.

The term "pharmaceutical composition" refers to a composition that is present in a form that allows the biological activity of the active ingredients contained therein to be effective, and which does not contain additional ingredients having unacceptable toxicity to the subject to which the composition is administered.

The term "effective amount" refers to an amount or dosage of a hybrid protein of the present invention or a nucleic acid encoding the same or a composition or combination thereof that, when administered to a patient in single or multiple doses, produces the desired effect in a patient in need of treatment or prevention.

A "therapeutically effective amount" refers to an amount effective, at dosages and for periods of time required, to achieve the desired therapeutic result. A therapeutically effective amount is also one in which any toxic or adverse effect of the hybrid protein or composition or combination is less than a therapeutically beneficial effect. A "therapeutically effective amount" preferably inhibits or improve a measurable parameter by at least about 40%, even more preferably at least about 50%, 55%, 60%, 65%, 70%, 75%, 80%, 85%, 90% or even 100% relative to untreated subjects.

A "prophylactically effective amount" refers to an amount effective, at dosages and for periods of time required, to achieve the desired prophylactic result. Generally, since a prophylactic dose is administered in a subject before or at an earlier stage of a disease, the prophylactically effective amount will be less than the therapeutically effective amount.

These and other aspects and embodiments of the present invention are described in the accompanying drawings (Brief Description of the Drawings immediately follows) and the following detailed description of the present invention and are illustrated in the following examples. Any or all of the features discussed above and throughout this application may be combined in various embodiments of the present invention. The following examples further illustrate the present invention; however, it should be understood that the examples have been described by way of illustration and not limitation, and that various modifications may be made by those skilled in the art.

## II. Hybrid protein

The present invention relates to a hybrid protein of CFH and DAF, which comprises at least one functional unit derived from CFH and at least one functional unit derived from DAF.

## A. DAF functional unit

In certain embodiments, the hybrid protein of the present invention preferably comprises a functional unit from DAF. Such functional units are capable of dissociating the C3 and C5 convertases and/or accelerating the decay-accelerating activity against C3 convertases in the classical pathway and/or in the alternative pathway. In some embodiments, the DAF functional unit comprises CCP4 of the DAF. In some embodiments, the DAF functional unit comprises CCP3 and CCP4 of DAF.

The amino acid sequence of such CCPs can be identical to the native or naturally occurring amino acid sequence of DAF. Alternatively, the amino acid sequence of such CCPs may be slightly altered, particularly at the amino or carboxy terminus. This alteration occurs when the restriction enzyme site is incorporated into the polynucleotide encoding the CCP. Such alterations also occur when an amino acid is deleted from the N-terminus or C-terminus of the functional unit. For example, in some embodiments, 1-2 amino acids may be deleted at the N-terminus of CCP3.

Some amino acid substitutions, preferably conservative substitutions, in the sequence, may also be introduced without affecting functional activity. Conservative substitutions may be made, for example, by substituting charged amino acids for one another or substituting hydrophilic amino acids for one another, hydrophobic amino acids for one another, and amino acids of similar mass for one another.

In some embodiments, the DAF functional unit comprises an amino acid sequence having at least 95%, 96%, 97%, 98%, or 99% identity to CCP4 of the human DAF protein. In some embodiments, the DAF functional unit comprises CCP4 of human DAF protein. In some embodiment, the DAF functional unit comprises an amino acid sequence having at least 95%, 96%, 97%, 98%, or 99% identity to CCP3 and/or 4 of the human DAF protein (or CCP3-4 directly linked together by the C-terminus of CCP3 and the N-terminus of CCP4). In some embodiments, the DAF functional unit comprises CCP3 and/or 4 of the human DAF protein (CCP3-4 directly linked together by the C-terminus of CCP3 and the N-terminus of CCP4). In some embodiments, the DAF functional unit comprises CCP3 and CCP4 of the human DAF protein. In some embodiments, the DAF function unit is CCP3-4, which is directly linked together by the C-terminus of CCP3 and the N-terminus of CCP4.

In some embodiments, the human DAF protein is a native human DAF protein. In some embodiments, the native human DAF protein comprises or consists of the amino acid sequence as set forth in SEQ ID NO: 1. DAF comprises or consists of the amino acid sequence encoded by the DNA sequence as set forth in SEQ ID NO: 2.

In some embodiments, CCP3 comprises or consists of amino acids 161-222 of DAF.

In some embodiments, CCP3 comprises or consists of the amino acid sequence as set forth in SEQ ID NO: 9, or an amino acid having at least 95%, 96%, 97%, 98%, or 99% identity to the amino acid sequence as set forth in SEQ ID NO: 9.

SEQ ID NO: 9: <u>KSCPNPGEIRNGOIDVPGGILFGATISFSCNTGYKLFGSTSSFCLISGSSVOWSDPLPECRE</u>

In some embodiments, CCP4 comprises or consists of amino acids 223-285 of DAF. In some embodiments, the C-terminus of CCP4 can be extended to contain additional 1-2 amino acids such as 1-2 contiguous amino acids for attachment of other functional units, for example, CCP4 comprises or consists of amino acids 223-287 of DAF.

In some embodiments, CCP4 comprises or consists of the amino acid sequence as set forth in SEQ ID NO: 10, or an amino acid sequence having at least 95%, 96%, 97%, 98%, or 99% identity to the amino acid sequence as set forth in SEQ ID NO: 10 or 11.

SEQ ID NO: 10:

IYCPAPPQIDNGIIQGERDHYGYRQSVTYACNKGFTMIGEHSIYCTVNNDEGEWSGPPPECRG.

SEQ ID NO: 11:

IYCPAPPQIDNGIIQGERDHYGYRQSVTYACNKGFTMIGEHSIYCTVNNDEGEWSGPPPECRGKS.

In some embodiments, CCP3-4 comprises or consists of amino acids 161-285 or amino acids 161-287 of DAF. In some embodiments, the DAF functional unit CCP3-4 comprises or consists of the amino acid sequence as set forth in SEQ ID NO: 12 or SEQ ID NO: 13, or an amino acid sequence having at least 95%, 96%, 97%, 98%, or 99% identity to the amino acid sequence as set forth in SEQ ID NO: 12 or SEQ ID NO: 13.

SEQ ID NO: 12: Human DAF CCP3-4

<u>KSCPNPGEIRNGQIDVPGGILFGATISFSCNTGYKLFGSTSSFCLISGSSVQWSDPLPECRE</u>
IYCPAPPQIDNGIIQGERDHYGYRQSVTYACNKGFTMIGEHSIYCTVNNDEGEWSGPPPECRG

SEQ ID NO: 13: Human DAF CCP3-4 (wherein C-terminus of CCP4 is extended by 2 additional amino acids)

KSCPNPGEIRNGQIDVPGGILFGATISFSCNTGYKLFGSTSSFCLISGSSVQWSDPLPECRE

IYCPAPPQIDNGIIQGERDHYGYRQSVTYACNKGFTMIGEHSIYCTVNNDEGEWSGPPPECRG

KS

When referring to the amino acid position of DAF, the amino acid sequence position corresponds to the amino acid position of SEQ ID NO: 1.

**B. CFH functional unit**

In certain embodiments, the hybrid protein of the present invention comprises functional units from CFH. This functional unit is capable of dissociating the alternative pathway C3 convertase C3bBb and/or binding to C3b. In a preferred embodiment, the CFH functional unit comprises CCPs 1 and 2 of CFH.

The amino acid sequence of the CCP of such CFH may be identical to the native or naturally occurring amino acid sequence of CFH. Alternatively, the amino acid sequence of such CCPs may be slightly altered, particularly at the amino or carboxy terminus. This alteration occurs when the restriction enzyme site is incorporated into the polynucleotide encoding the CCP. Such alteration also occurs when amino acids are deleted from or added to the N-terminus or C-terminus of the functional unit.

For example, in some embodiments, 1-2 amino acids may be deleted at the N-terminus of CCP1. In some embodiments, 1-2 amino acids may be deleted at the C-terminus of CCP2. In some embodiments, the N-terminus and/or C-terminus of CCP1 and/or CCP2 can also be extended, e.g., by the addition of 1-2 contiguous amino acids, for attachment of other functional units or sequences. Thus, terms "CCP1" and "CCP2" described herein encompass CCP1 and CCP2, respectively, with the addition of amino acids (e.g., 1-2 contiguous amino acids) at the N-terminus and/or C-terminus.

Some amino acid substitutions, preferably conservative substitutions, in the sequence, may also be introduced without affecting functional activity. Conservative substitutions may be made, for example, by substituting charged amino acids for one another or substituting hydrophilic amino acids for one another, hydrophobic amino acids for one another, and amino acids of similar mass for one another. For example, CCP1 of the CFH of the present invention can comprise a V62I mutation. For example, CCP2 of the CFH of the invention can comprise one, two, or three of E116T, Q119K, and V143E mutations.

In one embodiment, the CFH functional unit comprises an amino acid sequence having at least 95%, 96%, 97%, 98%, or 99% identity to CCP1 of the human CFH protein. In some embodiments, the CFH functional unit comprises CCP1 of the human CFH protein. In one embodiment, the CFH functional unit comprises an amino acid sequence having at least 95%, 96%, 97%, 98%, or 99% identity to CCP2 of the human CFH protein. In some embodiments, the CFH functional unit comprises CCP2 of the human CFH protein. In one embodiment, the CFH functional unit comprises an amino acid sequence having at least 95%, 96%, 97%, 98%, or 99% identity to CCP1 and/or 2 of the human CFH protein (or CCP1-2 directly linked together by the C-terminus of CCP1 and the N-terminus of CCP2). In some embodiments, the CFH functional unit comprises CCP1 and/or 2 of the human CFH protein (CCP1-2 linked directly together by the C-terminus of CCP1 and the N-terminus of CCP2). In some embodiments, the CFH functional unit comprises CCP1 and CCP2 of the human CFH protein. In some embodiments, the CFH function unit is CCP1-2, which is directly linked together by the C-terminus of CCP1 and the N-terminus of CCP2.

In some embodiments, the human CFH protein is a native human CFH protein. In some embodiments, the native human CFH protein comprises or consists of an amino acid sequence as set forth in SEQ ID NO: 3 or 5. In some embodiments, the CFH comprises or consists of the amino acid sequence encoded by the DNA sequence as set forth in SEQ ID NO: 4 or 6.

In some embodiments, CCP1 comprises or consists of amino acids 19-82 of CFH, optionally CCP1 comprises a V62I mutation. In some embodiments, CCP1 comprises or consists of the amino acid sequence as set forth in SEQ ID NO: 14 or SEQ ID NO: 15, or an amino acid sequence having at least 95%, 96%, 97%, 98%, or 99% identity to the amino acid sequence as set forth in SEQ ID NO: 14 or SEQ ID NO: 15, optionally CCP1 comprises a V62I mutation.

SEQ ID NO:14: Human CFH CCP1

EDCNELPPRRNTEILTGSWSDQTYPEGTQAIYKCRPGYRSLGNVIMVCRKGEWVALNPL

RKCQK

SEQ ID NO:15: Human CFH CCP1 (wherein CCP1 comprises V62I)

EDCNELPPRRNTEILTGSWSDQTYPEGTQAIYKCRPGYRSLGNIIMVCRKGEWVALNPL RKCQK

In some embodiments, CCP2 comprises or consists of amino acids 83-143 of CFH, optionally CCP2 comprises one, two, or three of E116T, Q119K, and V143E mutations. In some embodiments, CCP2 comprises or consists of the amino acid sequence as set forth in SEQ ID NO: 16 or SEQ ID NO: 17, or an amino acid sequence having at least 95%, 96%, 97%, 98%, or 99% identity to the amino acid sequence as set forth in SEQ ID NO: 16 or SEQ ID NO: 17, optionally CCP2 comprises one, two, or three of E116T, Q119K, and V143E mutations.

SEQ ID NO:16: Human CFH CCP2

RPCGHPGDTPFGTFTLTGGNVFEYGVKAVYTCNEGYQLLGEINYRECDTDGWTNDIPIC EV

SEQ ID NO:17: Human CFH CCP2, comprising E116T, Q119K, and V143E

RPCGHPGDTPFGTFTLTGGNVFEYGVKAVYTCN  GY  LLGEINYRECDTDGWTNDIPIC E

When referring to the amino acid position of CFH, the amino acid sequence position corresponds to the amino acid position of SEQ ID NO: 3.

In some embodiments, CCP1-2 comprises or consists of amino acids 19-143 of CFH, optionally CCP1-2 comprises one, two, three, or four of V62I, E116T, Q119K, and V143E mutations. In some embodiments, CCP1-2 comprises or consists of the amino acid sequence as set forth in SEQ ID NOs: 31-34, or an amino acid having at least 95%, 96%, 97%, 98%, or 99% identity to the amino acid sequence as set forth in SEQ ID NOs: 31-34.

>Human CFH CCP1-2 (**SEQ ID NO:31**)

EDCNELPPRRNTEILTGSWSDQTYPEGTQAIYKCRPGYRSLGNVIMVCRKGEWVALNPL RKCQKRPCGHPGDTPFGTFTLTGGNVFEYGVKAVYTCNEGYQLLGEINYRECDTDGWTNDI PICEV

>Human CFH CCP1-2, comprising a point mutation V62I (**SEQ ID NO:32**)

EDCNELPPRRNTEILTGSWSDQTYPEGTQAIYKCRPGYRSLGNIIMVCRKGEWVALNPL RKCQKRPCGHPGDTPFGTFTLTGGNVFEYGVKAVYTCN  GY  LLGEINYRECDTDGWTNDI PICE

>Human CFH CCP1-2, comprising three point mutations of E116T, Q119K, and V143E (**SEQ ID NO:33**)

EDCNELPPRRNTEILTGSWSDQTYPEGTQAIYKCRPGYRSLGNVIMVCRKGEWVALNPL RKCQKRPCGHPGDTPFGTFTLTGGNVFEYGVKAVYTCN  GY  LLGEINYRECDTDGWTNDI PICE

>Human CFH CCP1-2, comprising point mutations V62I, E116T, Q119K, and V143E (**SEQ ID NO:34**)

EDCNELPPRRNTEILTGSWSDQTYPEGTQAIYKCRPGYRSLGNIIMVCRKGEWVALNPL RKCQKRPCGHPGDTPFGTFTLTGGNVFEYGVKAVYTCN  GY  LLGEINYRECDTDGWTNDI PICE

**C. CR1 functional unit**

In certain embodiments, the hybrid protein of the present invention preferably comprises a functional unit from CR1. Such functional units are capable of dissociating the C3 and C5 convertases and/or accelerating the decay-accelerating activity against C3 convertases in the alternative pathway. In some embodiments, the CR1 functional unit comprises CCP15 or CCP8 of CR1.

The amino acid sequence of the CCP of such CR1 may be identical to the native or naturally occurring amino acid sequence of CR1. Alternatively, the amino acid sequence of such CR1 may be slightly altered, particularly at the amino or carboxy terminus. This alteration occurs when the restriction enzyme site is incorporated into the polynucleotide encoding the CCP. Such alterations also occur when amino acids are deleted from or added to the N-terminus or C-terminus of the functional unit.

For example, in some embodiments, 1-2 amino acids may be deleted at the N-terminus or C-terminus of CCP15 or CCP8. In some embodiments, the N-terminus or C-terminus of CCP15 or CCP8 can be extended by 1-2 amino acids, for example by means of adding additional contiguous amino acid residues.

Some amino acid substitutions, preferably conservative substitutions, may also be introduced into the sequence without affecting functional activities. Conservative substitutions may be made, for example, by substituting charged amino acids for one another or substituting hydrophilic amino acids for one another, hydrophobic amino acids for one another, and amino acids of similar mass for one another.

In one embodiment, the CR1 functional unit comprises an amino acid sequence having at least 95%, 96%, 97%, 98%, or 99% identity to CCP15 or CCP8 of the human CR1 protein. In some embodiments, the CR1 functional unit comprises CCP15 or CCP8 of the human CR1 protein.

In some embodiments, the human CR1 protein is a native human CR1 protein. In some embodiments, the native human CR1 protein comprises or consists of the amino acid sequence as set forth in SEQ ID NO: 7. In some embodiments, CR1 comprises or consists of the amino acid sequence that is encoded by the DNA sequence as set forth in SEQ ID NO: 8.

In some embodiments, CCP15 comprises or consists of amino acids 941-1001 of CR1, or CCP8 comprises or consists of amino acids 491-551 of CR 1.

In some embodiments, the CR1 functional unit comprises or consists of the amino acid sequence as set forth in SEQ ID NO: 18, or an amino acid having at least 95%, 96%, 97%, 98%, or 99% identity to the amino acid sequence as set forth in SEQ ID NO: 18.

SEQ ID NO:18: Human CR1 CCP15 or CCP8

<u>GHCQAPDHFLFAKLKTQTNASDFPIGTSLKYECRPEYYGRPFSITCLDNLVWSSPKDVC</u>

<u>KR</u>

When referring to the amino acid position of CR1, the amino acid sequence position corresponds to the amino acid position of SEQ ID NO: 7.

**D. Other units**

Optionally, the hybrid protein of the present invention may further comprise a tag, preferably of about 2-10 amino acids, added to the amino or carboxy terminus, e.g. the carboxy terminus, of the hybrid protein. Typically, such additions are made to stabilize the protein or to facilitate secretory expression or purification of the hybrid protein. Such tags are known in the art. Representative examples of such tags include sequences encoding a series of histidine residues (e.g., 2-10 histidines, e.g., 2, 3, 4, 5, 6, or 7 histidines), an epitope tag FLAG, herpes simplex glycoprotein D, beta-galactosidase, a maltose-binding protein, or glutathione S-transferase.

In some embodiments, the tag is a histidine residue, which may be added at the amino-terminus or the carboxy-terminus, e.g., the carboxy-terminus, of the hybrid protein.

In some embodiments, the tag is a 6×His tag of GHHHHHH (SEQ ID NO: 19).

Optionally, the hybrid protein of the present invention may also include a signal peptide, such as MGWSCIILFLVATATGVHS (SEQ ID NO: 20).

The present invention also encompasses hybrid proteins in which one or more amino acids are altered by post-translational processes or synthetic methods. Examples of such modifications include, but are not limited to, glycosylation, iodination, myristoylation, and PEGylation.

**E. Examples of hybrid proteins**

In some embodiments, the hybrid protein of the present invention comprises at least one functional unit from CFH and at least one functional unit from DAF.

In some embodiments, the hybrid protein of the present invention comprises CCP1 and CCP2 of CFH and CCP4 of DAF. In some embodiments, the hybrid proteins of the invention consist of CCP1 and CCP2 of CFH and CCP4 of DAF. In some embodiments, the hybrid protein of the present invention comprises or consists of CCP 1-2 of CFH and CCP4 of DAF.

In some embodiments, CCP1 of the CFH of the present invention can comprise a V62I mutation. In some embodiments, CCP2 of the CFH of the invention can comprise one, two, or three of E116T, Q119K, and V143E mutations.

In some embodiments, the hybrid protein of the present invention further comprises a signal peptide at the N-terminus, such as the amino acid sequence as set forth in SEQ ID NO: 20, and/or a tag at the C-terminus, such as a histidine tag, such as a 6×His-tag, such as GHHHHHH (SEQ ID NO: 19).

In some embodiments, the CCP4 of the DAF comprises or consists of amino acids 223-285 of the DAF protein, wherein the amino acid positions are numbered corresponding to the amino acid positions as set forth in SEQ ID NO: 1. In some embodiments, CCP4 of DAF is extended by 2 amino acids from its C-terminus, for example comprising or consisting of amino acids 223-287 of the DAF protein, wherein the amino acid positions are numbered corresponding to the amino acid positions as set forth in SEQ ID NO: 1.

In some embodiments, CCP1 of CFH comprises or consists of amino acids 19-82 of the CFH protein, wherein the amino acid positions are numbered corresponding to the amino acid positions as set forth in SEQ ID NO: 3, optionally CCP1 comprises a V62I mutation. In some embodiments, CCP2 of CFH comprises or consists of amino acids 83-143 of the CFH protein, wherein the amino acid positions are numbered corresponding to the amino acid positions as set forth in SEQ ID NO: 3, optionally CCP2 comprises one, two, or three of E116T, Q119K, and V143E mutations.

In some embodiments, CCP4 of DAF comprises or consists of amino acids 223-285 of the DAF protein, wherein the amino acid positions are numbered corresponding to the amino acid positions as set forth in SEQ ID NO: 1; and CCP1 of CFH comprises or consists of the amino acid sequence at positions 19-82 of the human CFH protein, CCP2 of the CFH comprises or consists of the amino acid sequence at positions 83-143 of the human CFH protein, wherein optionally the CCP1 comprises a V62I mutation, wherein optionally the CCP2 comprises one, two or three of E116T, Q119K, and V143E mutations, wherein the amino acid positions are numbered corresponding to the amino acid positions as set forth in SEQ ID NO: 3.

In some embodiments, CCP4 of DAF comprises or consists of amino acids 223-287 of the DAF protein, wherein the amino acid positions are numbered corresponding to the amino acid positions as set forth in SEQ ID NO: 1; and CCP1 of CFH comprises or consists of the amino acid sequence at positions 19-82 of the human CFH protein, CCP2 of the CFH comprises or consists of the amino acid sequence at positions 83-143 of the human CFH protein, wherein optionally the CCP1 comprises a V62I mutation, wherein optionally the CCP2 comprises one, two or three of E116T, Q119K and V143E mutations, wherein the amino acid positions are numbered corresponding to the amino acid positions as set forth in SEQ ID NO: 3.

In some embodiments, CCP4 of DAF comprises or consists of amino acids 223-285 of the DAF protein, wherein the amino acid positions are numbered corresponding to the amino acid positions as set forth in SEQ ID NO: 1; and CCP1-2 of CFH comprises or consists of the amino acid sequence at positions 19-143 of the human CFH protein, wherein optionally the CCP1-2 comprises one, two, three, or four of V62I, E116T, Q119K, and V143E mutations, wherein the amino acid positions are numbered corresponding to the amino acid positions as set forth in SEQ ID NO: 3.

In some embodiments, CCP4 of DAF comprises or consists of amino acids 223-287 of the DAF protein, wherein the amino acid positions are numbered corresponding to the amino acid positions as set forth in SEQ ID NO: 1; and the CCP1-2 of CFH comprises or consists of an amino acid sequence at positions 19-143 of the human CFH protein, wherein optionally the CCP1-2 comprises one, two, three or four of V62I, E116T, Q119K and V143E mutations, wherein the amino acid positions are numbered corresponding to the amino acid positions as set forth in SEQ ID NO: 3.

In some embodiments, the hybrid protein of the present invention comprises CCP15 or CCP8 of CR1 and CCP3 and CCP4 of DAF. In some embodiments, the hybrid protein of the invention consists of CCP15 or CCP8 of CR1 and CCP3 and CCP4 of DAF. In some embodiments, the hybrid protein of the present invention comprises or consists of CCP15 or CCP8 of CR1 and CCP3-4 of DAF.

In some embodiments, the hybrid protein of the present invention further comprises a signal peptide at the N-terminus, such as the amino acid sequence as set forth in SEQ ID NO: 13, and/or a tag at the C-terminus, such as a histidine tag, such as a 6×His-tag, such as GHHHHHH.

In some embodiments, CCP15 of CR1 comprises or consists of amino acids 941-1001 of the CFH protein, or CCP8 comprises or consists of amino acids 491-551 of the CFH protein, wherein the amino acid positions are numbered corresponding to the amino acid positions as set forth in SEQ ID NO: 3.

In some embodiments, CCP3 of DAF comprises or consists of amino acids 161-222 of the DAF protein, and/or CCP4 comprises or consists of amino acids 223-285 of the DAF protein, wherein the amino acid positions are numbered corresponding to the amino acid positions as set forth in SEQ ID NO: 1. In some embodiments, the CCP3-4 of DAF comprises or consists of amino acids 161-285 of the DAF protein, wherein the amino acid positions are numbered corresponding to the amino acid positions as set forth in SEQ ID NO: 1. In some embodiments, CCP4 of DAF is extended by 2 amino acids at its C-terminus, for example comprising or consisting of amino acids 223-287 of the DAF protein, and/or CCP3 comprises or consists of amino acids 161-222 of the DAF protein, wherein the amino acid positions are numbered corresponding to the amino acid positions as set forth in SEQ ID NO: 1. In some embodiments, CCP3-4 of DAF is extended by 2 amino acids at its C-terminus, for example, comprising or consisting of amino acids 161-287 of the DAF protein.

In some embodiments, CCP3-4 of DAF comprises or consists of amino acids 161-285 of the DAF protein, wherein the amino acid positions are numbered corresponding to the amino acid positions as set forth in SEQ ID NO: 1; and CCP15 of CR1 comprises or consists of amino acids 941-1001 of the CFH protein, or CCP8 comprises or consists of amino acids 491-551 of the CFH protein, wherein the amino acid positions are numbered corresponding to the amino acid positions as set forth in SEQ ID NO: 3.

In some embodiments, CCP3-4 of DAF comprises or consists of amino acids 161-287 of the DAF protein, wherein the amino acid positions are numbered corresponding to the amino acid positions as set forth in SEQ ID NO: 1; and CCP15 of CR1 comprises or consists of amino acids 941-1001 of the CFH protein, or CCP8 comprises or consists of amino acids 491-551 of the CFH protein, wherein the amino acid positions are numbered corresponding to the amino acid positions as set forth in SEQ ID NO: 3.

In some specific embodiments, the DAF is a human DAF protein, such as a native human DAF protein. In some specific embodiments, the DAF protein comprises or consists of

(i) the amino acid sequence as set forth in SEQ ID NO: 1;
(ii) the amino acid sequence encoded by the nucleic acid sequence as set forth in SEQ ID NO: 2;
(iii) the amino acid sequence having at least 95%, 96%, 97%, 98%, or 99% identity to the amino acid sequence as set forth in (i) or (ii); or

consists of the amino acid sequence as set forth in any one of (i) to (iii).

In some specific embodiments, CFH is a human CFH protein, such as a native human CFH protein. In some specific embodiments, the CFH protein comprises

(i) the amino acid sequence as set forth in SEQ ID NO: 3 or 5;
(ii) the amino acid sequence encoded by the nucleic acid sequence as set forth in SEQ ID NO: 4 or 6;
(iii) the amino acid sequence having at least 95%, 96%, 97%, 98%, or 99% identity to the amino acid sequence as set forth in (i) or (ii); or

consists of the amino acid sequence as set forth in any one of (i) to (iii).

In some specific embodiments, CR1 is a human CR1 protein, such as a native human CR1 protein. In some specific embodiments, the CR1 protein comprises

(i) the amino acid sequence as set forth in SEQ ID NO: 7;
(ii) the amino acid sequence encoded by the nucleic acid sequence as set forth in SEQ ID NO: 8;
(iii) the amino acid sequence having at least 95%, 96%, 97%, 98%, or 99% identity to the amino acid sequence as set forth in (i) or (ii); or

consists of the amino acid sequence as set forth in any one of (i) to (iii).

In some embodiments, the hybrid protein of the present invention comprises or consists of the amino acid sequence of any one of SEQ ID NOs: 21-28, or an amino acid sequence having at least 95%, 96%, 97%, 98%, or 99% identity to the amino acid sequence.

>Human CFH CCP1-2 or SCR1-2 - Human DAF/CD55 CCP4 or SCR4: **(SEQ ID NO:21)**

EDCNELPPRRNTEILTGSWSDQTYPEGTQAIYKCRPGYRSLGNVIMVCRKGEWVALNPL
RKCQKRPCGHPGDTPFGTFTLTGGNVFEYGVKAVYTCNEGYQLLGEINYRECDTDGWTNDI
PICEVIYCPAPPQIDNGIIQGERDHYGYRQSVTYACNKGFTMIGEHSIYCTVNNDEGEWSGPPP
ECRG

>Human CFH CCP1-2 or SCR1-2 - Human DAF/CD55 CCP4 or SCR4: **(SEQ ID NO:22)**

EDCNELPPRRNTEILTGSWSDQTYPEGTQAIYKCRPGYRSLGN**I**IMVCRKGEWVALNPL
RKCQKRPCGHPGDTPFGTFTLTGGNVFEYGVKAVYTCN GY LLGEINYRECDTDGWTNDI
PICE IYCPAPPQIDNGIIQGERDHYGYRQSVTYACNKGFTMIGEHSIYCTVNNDEGEWSGPPP
ECRG

Wherein the CFH CCP1/SCR1 region comprises a point mutation V62I **(bold type)**

>Human CFH CCP1-2 or SCR1-2 - Human DAF/CD55 CCP4 or SCR4: **(SEQ ID NO:23)**

EDCNELPPRRNTEILTGSWSDQTYPEGTQAIYKCRPGYRSLGN**V**IMVCRKGEWVALNPL
RKCQKRPCGHPGDTPFGTFTLTGGNVFEYGVKAVYTCN GY LLGEINYRECDTDGWTNDI
PICE IYCPAPPQIDNGIIQGERDHYGYRQSVTYACNKGFTMIGEHSIYCTVNNDEGEWSGPPP
ECRG

Wherein the CFH CCP2/SCR2 region comprises three point mutations of E116T, Q119K, and V143E **(bold type)**

>Human CFH CCP1-2 or SCR1-2 - Human DAF/CD55 CCP4 or SCR4: **(SEQ ID NO:24)**

EDCNELPPRRNTEILTGSWSDQTYPEGTQAIYKCRPGYRSLGN**I**IMVCRKGEWVALNPL
RKCQKRPCGHPGDTPFGTFTLTGGNVFEYGVKAVYTCN GY LLGEINYRECDTDGWTNDI
PICE IYCPAPPQIDNGIIQGERDHYGYRQSVTYACNKGFTMIGEHSIYCTVNNDEGEWSGPPP
ECRG

Wherein the CFH CCP1/SCR1 region comprises a point mutation V62I **(bold type)**
Wherein the CFH CCP2/SCR2 region comprises three point mutations of E116T, Q119K, and V143E **(bold type)**

>Human CFH CCP1-2 or SCR1-2 - Human DAF/CD55 CP4 or SCR4: **(SEQ ID NO:25)**

EDCNELPPRRNTEILTGSWSDQTYPEGTQAIYKCRPGYRSLGN**V**IMVCRKGEWVALNPL
RKCQKRPCGHPGDTPFGTFTLTGGNVFEYGVKAVYTCN GY LLGEINYRECDTDGWTNDI
PICE IYCPAPPQIDNGIIQGERDHYGYRQSVTYACNKGFTMIGEHSIYCTVNNDEGEWSGPPP
ECRGKS*GHHHHHH*

A 6×His tag is attached, and wherein the DAF CCP4 is extended by two amino acid residues in the C-terminal direction

>Human CFH CCP1-2 or SCR1-2 - Human DAF/CD55 CCP4 or SCR4: **(SEQ ID NO:26)**

EDCNELPPRRNTEILTGSWSDQTYPEGTQAIYKCRPGYRSLGN**I**IMVCRKGEWVALNPL
RKCQKRPCGHPGDTPFGTFTLTGGNVFEYGVKAVYTCN GY LLGEINYRECDTDGWTNDI
PICE IYCPAPPQIDNGIIQGERDHYGYRQSVTYACNKGFTMIGEHSIYCTVNNDEGEWSGPPP
ECRGKS*GHHHHHH*

A 6×His tag is attached, and wherein the DAF CCP4 is extended by two amino acid residues in the C-terminal direction

Wherein the CFH CCP1/SCR1 region comprises a point mutation V62I (**bold type**)

>Human CFH CCP1-2 or SCR1-2 - Human DAF/CD55 CCP4 or SCR4: **(SEQ ID NO:27)**

EDCNELPPRRNTEILTGSWSDQTYPEGTQAIYKCRPGYRSLGN**V**IMVCRKGEWVALNPL RKCQKRPCGHPGDTPFGTFTLTGGNVFEYGVKAVYTCN GY LLGEINYRECDTDGWTNDI PICE IYCPAPPQIDNGIIQGERDHYGYRQSVTYACNKGFTMIGEHSIYCTVNNDEGEWSGPPP ECRGKS*GHHHHHH*

A 6×His tag is attached, and wherein the DAF CCP4 is extended by two amino acid residues in the C-terminal direction

Wherein the CFH CCP2/SCR2 region comprises three point mutations of E116T, Q119K, and V143E (**bold type**)

>Human CFH CCP1-2 or SCR1-2 - Human DAF/CD55 CCP4 or SCR4: **(SEQ ID NO:28)**

EDCNELPPRRNTEILTGSWSDQTYPEGTQAIYKCRPGYRSLGN**I**IMVCRKGEWVALNPL

RKCQKRPCGHPGDTPFGTFTLTGGNVFEYGVKAVYTCN GY LLGEINYRECDTDGWTNDI PICE IYCPAPPQIDNGIIQGERDHYGYRQSVTYACNKGFTMIGEHSIYCTVNNDEGEWSGPPP ECRGKS*GHHHHHH*

A 6×His tag is attached, and wherein the DAF CCP4 is extended by two amino acid residues in the C-terminal direction
Wherein the CFH CCP1/SCR1 region comprises a point mutation V62I (**bold type**)
Wherein the CFH CCP2/SCR2 region comprises three point mutations of E116T, Q119K, and V143E (**bold type**)

In some embodiments, the hybrid protein of the present invention comprises or consists of the amino acid sequence as set forth in SEQ ID NO: 29 or 30, or an amino acid sequence having at least 95%, 96%, 97%, 98%, or 99% identity to the amino acid sequence.

>Human CR1 CCP15 or SCR15 - Human DAF/CD55 CCP3-4 or SCR3-4: (SEQ ID NO: 29)

GHCQAPDHFLFAKLKTQTNASDFPIGTSLKYECRPEYYGRPFSITCLDNLVWSSPKDVC KRKSCPNPGEIRNGQIDVPGGILFGATISFSCNTGYKLFGSTSSFCLISGSSVQWSDPLPECREI YCPAPPQIDNGIIQGERDHYGYRQSVTYACNKGFTMIGEHSIYCTVNNDEGEWSGPPPECRG

>Human CR1 CCP15 or SCR15 - Human DAF/CD55 CCP3-4 or SCR3-4: (SEQ ID NO: 30)

GHCQAPDHFLFAKLKTQTNASDFPIGTSLKYECRPEYYGRPFSITCLDNLVWSSPKDVC KRKSCPNPGEIRNGQIDVPGGILFGATISFSCNTGYKLFGSTSSFCLISGSSVQWSDPLPECREI YCPAPPQIDNGIIQGERDHYGYRQSVTYACNKGFTMIGEHSIYCTVNNDEGEWSGPPPECRG KS*GHHHHHH*

A 6×His tag is attached, and wherein the DAF CCP4 is extended by two amino acid residues in the C-terminal direction

## III. Polynucleotides, vectors and hosts

The present invention provides a nucleic acid encoding any of the above hybrid proteins of the present invention. Also provided is a vector comprising the nucleic acid. In one embodiment, the vector is an expression vector (e.g., a pcDNA vector, such as pcDNA3.1). Also provided is a host cell comprising the nucleic acid or the vector. In one embodiment, the host cell is eukaryotic. In another embodiment, the host cell is selected from a yeast cell or a mammalian cell (e.g., CHO cells or 293 cells, such as Expi293 cells). In another embodiment, the host cell is prokaryotic.
In one aspect, the present invention provides a nucleic acid encoding any of the above hybrid proteins. The polypeptide encoded by the nucleic acid is capable of having DAF and/or CFH function when expressed from a suitable expression vector, e.g., dissociating C3 and/or C5 convertase, accelerating decay-accelerating activity against the classical pathway C3 convertase and/or the alternative pathway C3 convertase, dissociating the alternative pathway C3 convertase C3bBb, or binding to C3b, and biased complement-inhibiting activity, including activity inhibiting the Alternative Pathway (AP), but substantially not inhibiting the Classical Pathway (CP).

For ease of production and purification, the hybrid protein may be fused at the N-terminus to a secretory signal peptide, and/or a tag peptide, such as a hexahistidine tag or biotin tag, to facilitate the purification.

As will be appreciated by those skilled in the art, each hybrid protein may be encoded by a variety of nucleic acid sequences due to codon degeneracy.

In some embodiments, the nucleic acid of the present invention comprises a nucleic acid encoding the amino acid sequence selected from any one of SEQ ID NOs: 21-30, or an amino acid sequence having at least 85%, 90%, 91%, 92%, 93%, 94%, 95%, 96%, 97%, 98%, or 99% identity to the amino acid sequence selected from any one of SEQ ID NOs: 21-30.

Nucleic acid sequences encoding the molecules of the present invention may be generated using methods well known in the art, for example by de novo solid-phase DNA synthesis, or by PCR amplification.

In one embodiment, one or more vectors comprising a nucleic acid of the present invention are provided. In one embodiment, the vector is an expression vector, such as a prokaryotic expression vector or a eukaryotic expression vector. The vector includes, but are not limited to, viruses, plasmids, cosmids, Lambda phages or yeast YACs. In a preferred embodiment, the expression vector is a pcDNA, such as pcDNA3.1.

In one embodiment, a host cell comprising one or more polynucleotides of the present invention is provided. In some embodiments, a host cell comprising an expression vector of the present invention is provided. As used herein, the term "host cell" refers to any kind of cellular system that can be engineered to produce a protein of the present invention. Host cells suitable for replicating and supporting the expression of the proteins of the present invention are well-known in the art. Such cells can be transfected or transduced with a particular expression vector as desired, and large numbers of cells comprising the vector can be cultivated for seeding large-scale fermenters to obtain sufficient quantities of the protein of the present invention for clinical use.

Suitable host cells include prokaryotic microorganisms such as E.coli, eukaryotic microorganisms such as filamentous fungi or yeast, or various eukaryotic cells such as Chinese hamster ovary Cells (CHO), insect cells, and the like. Mammalian cell lines suitable for suspension culture may be used. Examples of useful mammalian host cell lines include SV40 transformed monkey kidney CV1 line (COS-7); human embryonic kidney lines (HEK 293 or 293F cells or 293FT cells), baby hamster kidney cells (BHK), monkey kidney cells (CV1), Vero cells (VERO-76), human cervical cancer cells (HELA), canine kidney cells (MDCK), Buffalo rat liver cells (BRL 3A), human lung cells (W138), human liver cells (Hep G2), CHO cells, NSO cells, myeloma cell lines such as YO, NS0, P3X63, and Sp2/0, and the like. Mammalian host cell lines suitable for the production of proteins are known in the art. In a preferred embodiment, the host cell is a CHO or HEK293 cell or an Expi293 cell.

## IV. Production and Purification of Molecules of the Invention

In yet another aspect, the present invention provides a method for producing a hybrid protein of the present invention, the method comprising: culturing a host cell comprising a nucleic acid molecule encoding the protein under conditions suitable for expression of the hybrid protein, optionally further comprising isolating the protein from the host cell or the host cell culture medium, and/or purifying the protein.

For recombinant production, a polynucleotide encoding a protein of the present invention may be inserted into a vector for further cloning and/or expression in a host cell. Methods well known to those skilled in the art can be used to construct expression vectors. Once an expression vector comprising a polynucleotide of the present invention has been prepared for expression, the expression vector can be transfected or introduced into a suitable host cell. Various techniques can be used to achieve this purpose, for example, protoplast fusion, calcium phosphate precipitation, electroporation, retroviral transduction, viral transfection, biolistics, liposome-based transfection, or other conventional techniques.

Proteins prepared as described herein can be purified by known prior art techniques such as nickel column, high-performance liquid chromatography, affinity chromatography, ion exchange chromatography, gel electrophoresis, and the like. The actual conditions used to purify a particular protein also depend on factors such as net charge, hydrophobicity, hydrophilicity, and the like, and will be apparent to those skilled in the art.

The purity and quantification of the proteins of the present invention can be determined by any of a variety of well-known analytical methods.

## V. Assay method

The hybrid proteins provided herein can be identified, screened, or characterized for their physical/chemical properties and/or biological activity by a variety of assays known in the art.

The hemolytic inhibitory effect of the hybrid protein of the present invention can be measured by methods known in the art, such as in vitro assays and/or in vivo animal experiments. For example, a hemolytic assay, such as the method described in Examples 4.1 and 4.2, can be used to test for hemolytic inhibitory effects of the molecule on the classical and/or alternative pathways of complement immunity.

## VI. Pharmaceutical compositions, pharmaceutical combinations, and kits

In one aspect, the present invention provides a composition, e.g., a pharmaceutical composition, medicament, or formulation, comprising a hybrid protein of the present invention or a nucleic acid molecule encoding the same.

In one embodiment, the composition further comprises a pharmaceutical excipient, such as a pharmaceutical carrier, a pharmaceutical excipient, including buffers, as known in the art.

As used herein, "pharmaceutically acceptable carrier" includes any and all solvents, dispersion media, isotonic agent and absorption delaying agents, and the like that are physiologically compatible. See also "Handbook of Pharmaceutical Excipients", 8th Edition, R.C.Rowe, P.J. Seskey and S.C. Owen, Pharmaceutical Press, London, Chicago for the use of pharmaceutically acceptable auxiliary materials and uses thereof.

The compositions or medicaments or formulations of the present invention may be in a variety of forms. These forms include, for example, liquid, semi-solid, and solid dosage forms, such as liquid solutions (e.g., injectable solutions), powders or suspensions, liposomal formulations, and suppositories. The preferred form depends on the intended mode of administration and therapeutic use.

In addition to the hybrid protein of the present invention or the nucleic acid encoding the same, the compositions or medicaments or formulations of the present invention may also comprise additional therapeutic agents required for the particular indication being treated, preferably those that do not adversely affect the activity of each other. Thus, in one embodiment, a composition or formulation or medicament, e.g., a pharmaceutical composition, comprises a combination of one or more molecules of the present invention, and one or more additional therapeutic agents.

The compositions or medicaments or formulations of the present invention may be in a variety of forms. These forms include, for example, liquid, semi-solid, and solid dosage forms, such as liquid solutions (e.g., injectable solutions), powders or suspensions, liposomal formulations, and suppositories. The compositions or medicaments or formulations of the present invention are suitable for administration by intravenous bolus injection, intravenous infusion, intraperitoneal, intradermal, intramuscular, subcutaneous, and intranasal routes (e.g., by injection or infusion). The preferred form depends on the intended mode of administration and therapeutic use.

The present invention also provides a pharmaceutical combination or a pharmaceutical combination product comprising a hybrid protein of the present invention or a nucleic acid encoding the same and one or more other therapeutic agents.

The present invention also provides a kit of parts comprising the pharmaceutical combination, e.g., the kit of parts comprises the following in the same package:

- a first container comprising a pharmaceutical composition comprising a hybrid protein of the present invention or a nucleic acid encoding the same; and
- optionally, a second container comprising a pharmaceutical composition comprising one or more additional therapeutic agents (in some embodiments, the two or more additional therapeutic agents are in the same container, or in separate containers).

## VII. Uses and Methods

In one aspect of the present invention, provided is a method of preventing or treating a complement system-associated disease or disorder in a subject, comprising administering to the subject an effective amount of the hybrid protein or the nucleic acid encoding the same of the present invention or the composition or medicament or formulation comprising the protein.

In some embodiments, the complement system-associated disease is due to abnormal activation of the complement system or a dysregulation of the complement system, particularly of the complement alternative pathway. In some embodiments, the abnormal activation of the complement system or dysregulation of the complement system, particularly of the complement alternative pathway, is due to, for example, microbial infection or an increase in autoimmune antibodies, or to a decrease, deletion, incapacitation, or interference or blockade of a function of complement regulatory proteins, etc.

In some embodiments, the disease treatment will benefit from inhibiting the activity of the complement system, particularly inhibiting the complement alternative pathway biasedly.

In other aspects, the present invention provides the use of the molecule of the present invention, or a composition comprising the same, in the manufacture or preparation of a medicament for use as described herein, for example, for the prevention or treatment of a complement system-associated disease or disorder as mentioned herein, for example a disease or disorder associated with abnormal activation or dysregulation of the complement alternative pathway.

In some embodiments, the complement system-associated disease or disorder can be a disease requiring hemolysis inhibition, e.g., a disease requiring inhibition of hemolysis of the classical pathway of the complement immunity and/or the alternative pathway of the complement immunity.

In some embodiments, the complement system-associated disease or disorder is a disease in which inhibition of complement alternative pathway activity is desired, such as Dense Deposition Disease (DDD), age-related macular degeneration (AMD), membranoproliferative glomerulonephritis type II (MPGN II), hemolytic uremic syndrome (HUS), asthma, amyloidosis, glomerulonephritis (e.g., immune complex glomerulonephritis), membranous nephritis, transplant rejection, antiphospholipid antibody syndrome, pemphigus, intestinal inflammation, lupus nephritis, eye drop-related diseases, or thrombotic thrombocytopenic purpura.

In some preferred embodiments, the complement system-associated disease or disorder is a disease in which inhibition of complement alternative pathway activity while retaining of complement classical pathway activity is desired, such as Dense Deposition Disease (DDD), age-related macular degeneration (AMD), membranoproliferative glomerulonephritis type II (MPGN II), hemolytic uremic syndrome (HUS), asthma, amyloidosis, glomerulonephritis (e.g., immune complex glomerulonephritis), membranous nephritis, transplant rejection, antiphospholipid antibody syndrome, pemphigus, intestinal inflammation, lupus nephritis, eye drop-related diseases, or thrombotic thrombocytopenic purpura.

In some embodiments, the hybrid protein or the nucleic acid encoding the same of the present invention or the composition or medicament or formulation comprising the proteins or nucleic acids delays the onset of a disorder and/or a symptom associated with the disorder.

In some embodiments, the hybrid protein or the nucleic acid encoding the same of the present invention or the composition or medicament or formulation comprising the proteins or nucleic acids can also be administered in combination with one or more other therapies, e.g., therapeutic modalities and/or other therapeutic agents, for use as described herein, e.g., for the prevention and/or treatment of the related diseases or disorders as mentioned herein.

The routes of administration of the hybrid protein or the nucleic acid encoding the same of the present invention or the composition or medicament or formulation comprising the proteins or nucleic acids depend on known methods, e.g., injection or infusion.

The invention further encompasses the use of the hybrid protein or the nucleic acid encoding the same of the present invention or the composition or formulation comprising the proteins or nucleic acids in the manufacture of a medicament for use as described herein, e.g., for the prevention and/or treatment of a related disease or disorder as mentioned herein.

**Examples**

**Example 1 Molecular construction**

In order to construct the protein expression vector of the hybrid molecule involved in the present invention and the complement regulatory molecule of the prior art, the DNA encoding each protein was subcloned into the pcDNA3.1 expression vector (purchased from Biofeng) with the synthesized gene DNA (GENEWIZ Inc., Suzhou/Sangon Biotech (Shanghai) Co., Ltd.) as a template by using a method of conventional molecular cloning technology. The plasmid was verified by sequencing and then was used for transient protein expression. A signal peptide MGWSCIILFLVATATGVHS was fused to the N-terminus of each encoded protein, while a 6×His tag (GHHHHHH) attached to the C-terminus, so that subsequently the proteins could be expressed in a secreted form with mammalian cells and be purified by nickel column.

The amino acid sequence of each encoded protein is as follows:

>CR13m (human CR1 CCP1-3 or SCR1-3, comprising N29K, S37Y, G79D, and D109N mutations (**bold type**), the sequence is derived from patent US9988611_B2): SEQ ID NO: 35

QCNAPEWLPFARPTNLTDEFEFPIGTYL**K**YECRPGYY**Y**GRPFSIICLKNSVWTGAKDRCRR KSCRNPPDPVNGMVHVIK**D**IQFGSQIKYSCTKGYRLIGSSSATCIISG**N**TVIWDNETPICDRIPC GLPPTITNGDFISTNRENFHYGSVVTYRCNPGSGGRKVFELVGEPSIYCTSNDDQVGIWSGPA PQCI*GHHHHHH*

>D24 (human DAF/CD55 CCP2-4 or SCR2-4, the sequence references Hui-fen Zhang et al., THE JOURNAL OF BIOLOGICAL CHEMISTRY, Vol. 276, No. 29, Issue of July 20, pp. 27290-27295, 2001): SEQ ID NO: 36

SCEVPTRLNSASLKQPYITQNYFPVGTVVEYECRPGYRREPSLSPKLTCLQNLKWSTAVE
FCKKKSCPNPGEIRNGQIDVPGGILFGATISFSCNTGYKLFGSTSSFCLISGSSVQWSDPLPECR
EIYCPAPPQIDNGIIQGERDHYGYRQSVTYACNKGFTMIGEHSIYCTVNNDEGEWSGPPPECR
G*GHHHHHHH*

>F15 (human CFH CCP1-5 or SCR1-5, comprising a V62I mutation (bold type), the sequence references Masha Fridkis-Hareli et al., Blood. 2011 Oct 27; 118(17): 4705-4713 and Agustín Tortajada et al., Hum Mol Genet. 2009 September 15; 18(18):3452-3461): SEQ ID NO: 37

EDCNELPPRRNTEILTGSWSDQTYPEGTQAIYKCRPGYRSLGN IMVCRKGEWVALNPL
RKCQKRPCGHPGDTPFGTFTLTGGNVFEYGVKAVYTCNEGYQLLGEINYRECDTDGWTNDI
PICEVVKCLPVTAPENGKIVSSAMEPDREYHFGQAVRFVCNSGYKIEGDEEMHCSDDGFWSK
EKPKCVEISCKSPDVINGSPISQKIIYKENERFQYKCNMGYEYSERGDAVCTESGWRPLPSCE

EKSCDNPYIPNGDYSPLRIKHRTGDEITYQCRNGFYPATRGNTAKCTSTGWIPAPRCTLKP*GH
HHHHH*

>F12D4 (a hybrid molecule of the present invention, <u>human CFH CCP1-2 or SCR 1-2</u> -Human DAF/CD55 CCP4 or SCR4, wherein the CFH CCP2/SCR2 region comprises three point mutations (bold type) and the mutation amino acids are derived from the corresponding amino acid residues of DAF CCP3/SCR3) SEQ ID NO: , the sequence with a signal peptide connected to the N terminus is SEQ ID NO: 38

<u>EDCNELPPRRNTEILTGSWSDQTYPEGTQAIYKCRPGYRSLGNIIMVCRKGEWVALNPL
RKCQKRPCGHPGDTPFGTFTLTGGNVFEYGVKAVYTCN GY LLGEINYRECDTDGWTNDI
PICE IYCPAPPQIDNGIIQGERDHYGYRQSVTYACNKGFTMIGEHSIYCTVNNDEGEWSGPPP
ECRGKS</u>*GHHHHHH*

>CR15D34 (a hybrid molecule of the present invention, <u>human CR1 CCP15 or SCR15</u> - <u>human DAF/CD55 CCP3-4 or SCR3-4</u>): SEQ ID NO: 39

<u>GHCQAPDHFLFAKLKTQTNASDFPIGTSLKYECRPEYYGRPFSITCLDNLVWSSPKDVC
KRKSCPNPGEIRNGQIDVPGGILFGATISFSCNTGYKLFGSTSSFCLISGSSVQWSDPLPECREI
YCPAPPQIDNGIIQGERDHYGYRQSVTYACNKGFTMIGEHSIYCTVNNDEGEWSGPPPECRG</u>
*GHHHHHH*

>FH13 (human CFH CCP1-3 or SCR1-3): SEQ ID NO: 40

EDCNELPPRRNTEILTGSWSDQTYPEGTQAIYKCRPGYRSLGNIIMVCRKGEWVALNPL
RKCQKRPCGHPGDTPFGTFTLTGGNVFEYGVKAVYTCNEGYQLLGEINYRECDTDGWTNDI
PICEVVKCLPVTAPENGKIVSSAMEPDREYHFGQAVRFVCNSGYKIEGDEEMHCSDDGFWSK
EKPKCVEIS*GHHHHHH*

>D14 (human DAF/CD55 CFH CCP1-4 or SCR1-4) :SEQ ID NO: 41

DCGLPPDVPNAQPALEGRTSFPEDTVITYKCEESFVKIPGEKDSVICLKGSQWSDIEEFCN
RSCEVPTRLNSASLKQPYITQNYFPVGTVVEYECRPGYRREPSLSPKLTCLQNLKWSTAVEFC
KKKSCPNPGEIRNGQIDVPGGILFGATISFSCNTGYKLFGSTSSFCLISGSSVQWSDPLPECREI
YCPAPPQIDNGIIQGERDHYGYRQSVTYACNKGFTMIGEHSIYCTVNNDEGEWSGPPPECRG
KS*GHHHHHHH*

## Example 2. Protein expression

After amplification of the plasmid with strain DH5 alpha (Yeasen Biotechnology), the plasmid was prepared using the NucleoBond Xtra Midi Plus (MACHEREY-NAGEL, see the product Manual for details). Expi293 cells (purchased from Thermo fisher) were transfected with the prepared plasmids using PEI (Polyscience) for transient expression of proteins (see Jäger, V., et al., BMC Biotechnol 13, 52, 2013. for the transfection method). The harvested supernatant was purified by affinity chromatography in a nickel ion pre-packed column (GE life sciences, HisTrap HP, see product Manual for details), dialyzed against PBS for fluid changes, the fluid change protein samples were sterilized by filtration through 0.2 μm filters, and protein quantification was performed according to the theoretical extinction coefficient using the NanoDrop (Thermofisiher) A280 method.

## Example 3. Protein purity detection

### SDS-PAGE

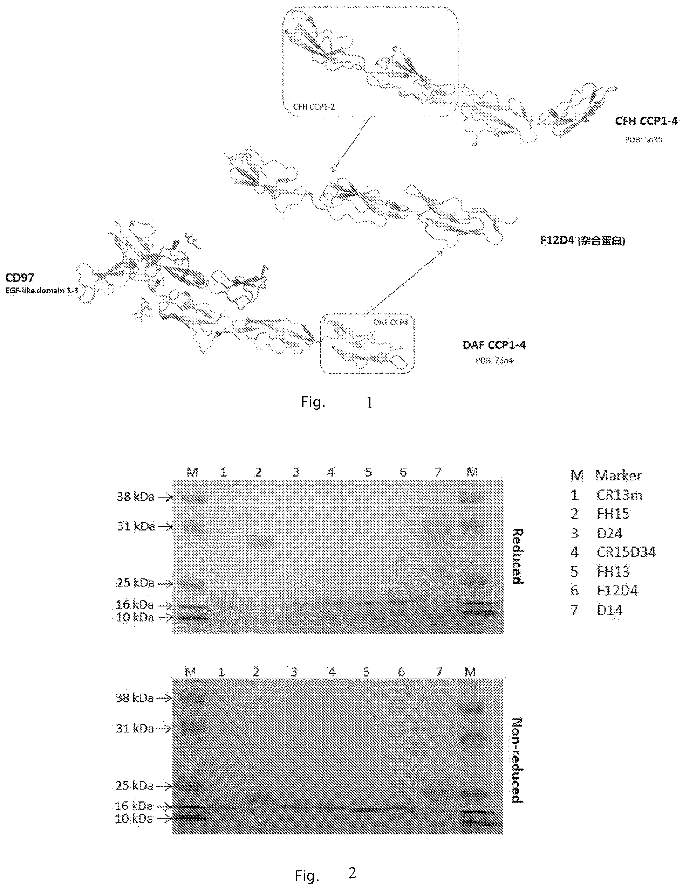

2.5 μg of protein was each taken and added proportionally to the loading buffer (Sangon Biotech) with reducing agent and without reducing agent, respectively. The samples with loading buffer containing reducing agent were boiled at 95 °C for 5 minutes to fully denature the proteins. Electrophoresis was performed with a 10% Precast-GLgel Tris-Glycine precast gel (Sangon Biotech) in a Mini-PROTEAN® Tetra electrophoresis system (BioRad). After completing the electrophoresis, the gel was stained with 0.1% Coomassie Brilliant Blue staining solution and de-stained with an ethanol-glacial acetic acid solution. The SDS-PAGE electrophoresis results showed that except for a slight diffusion of the D14 protein band, the other protein bands were relatively clear, indicating that the purity of the proteins under both reducing and non-reducing conditions was good (as shown in Figure 2). Additionally, due to differences in protein reduction, denaturation, modification status, and electrophoresis conditions, there was a certain discrepancy between the electrophoretic molecular weight and the theoretical molecular weight.

### HPLC-SEC

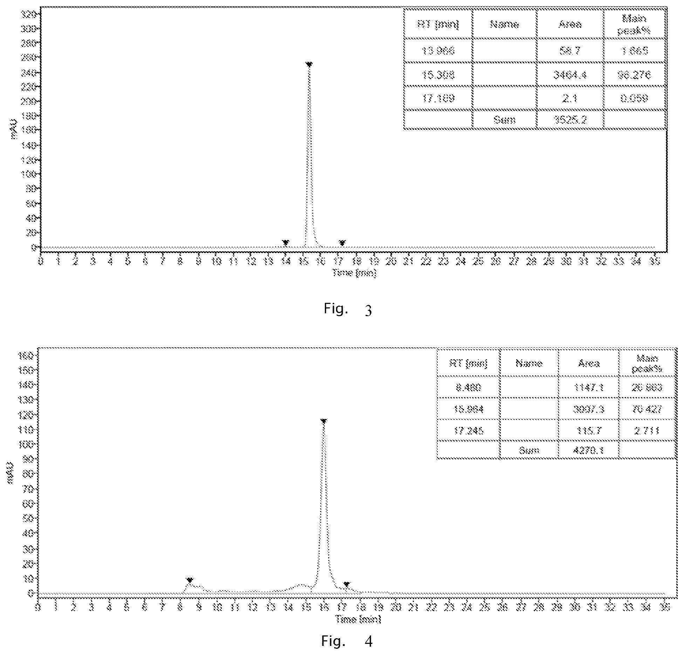

Approximately 50 μg of the purified protein sample was applied to a TSKgelSuper SW3000 column (5 μm) of 300×4.6mm (TOSOH). SEC detection was performed using a U3000 HPLC instrument (DIONEX). All proteins were detected using UV at 280 nm and 214 nm. Isocratic elution was performed at a flow rate of 0.25 mL/min. The analysis result showed that the hybrid protein F12D4 purified by a nickel column in one step had excellent purity, with a monomer purity of more than 98%, while the hybrid protein CR15D34 had good purity, with a monomer purity of more than 70% and a larger number of protein aggregates (as shown in figures 3 and 4).

## Example 4. Detection of complement-inhibiting activity

4.1 Detection of inhibitory activity on Classical Pathway of complement (CP)

In this experiment, the degree of inhibition of hemolysin-mediated hemolysis of sheep red blood cells caused by normal human serum complement activity was measured by testing the protein to be tested at different concentrations, and the inhibitory activity of each protein on complement CP was analyzed and compared.

The experimental process was briefly described as follows:

(1) Gradient dilution of proteins: the protein was formulated into a 12000 nM solution with GVB++ buffer (Gelatin Flora Buffer comprising Ca2+ and Mg2+, Cat. No.: 25-02080, TIANDZ), followed by 4-fold gradient dilution, with a total of 7 gradients;

(2) Dilution of serum: The normal human serum NHS (Shanghai Schbio Co., Ltd.) was taken out of the -70°C refrigerator, followed by naturally thawing at 4°C and then the serum was diluted to 4% with GVB++ buffer;

(3) Activation of sheep red blood cells: the sheep red blood cells (Nanjing SenBeiJia Biological Technology Co., Ltd.)

were washed with GVB++ buffer until the supernatant was transparent, then resuspended with GVB++ buffer, and hemolysin (BM351Y, Beijing Bersee Technology Co., Ltd.) was added to the red blood cell suspension at a ratio of 1:4000, followed by incubation at 4°C for 15 minutes to activate the sheep red blood cells. The activated sheep red blood cells were washed twice with GVB++ and resuspended in GVB++ buffer;

(4) Incubation: 25 μL each of the diluted protein and serum were mixed in a 96-well plate, then 50 μL of the red blood cell suspension obtained in step (3) was added, resulting in an initial final concentration of the protein of 3000 nM and a final concentration of the serum of 1%. The mixture was blended thoroughly and placed in a 37°C constant temperature incubator for 1 hour, with a negative control (comprising only serum) and a positive control (comprising only serum and red blood cells);

(5) Termination: After the incubation, 100 μL of 20 mM EDTA-GVB buffer (GVBE) was added to each reaction well to terminate the reaction;

(6) Reading value: The 96-well plate was centrifuged at 3000 rpm for 5 minutes. 100 μL of the supernatant was added into a new 96-well flat-bottom plate. The absorbance value at OD405nm was measured in a multi-function plate reader, and the data were saved;

(7) Data processing: the obtained OD405nm reading value was put into the following formula to calculate the red blood cell hemolysis inhibition rate:

Hemolysis inhibition rate (%) = (positive control reading value - experimental group reading value) / (positive control reading value - negative control reading value) * 100%

Then the IC50 value of the measured inhibitory protein was calculated by plotting a four-parameter fit curve, with the final protein concentration as the abscissa and the hemolysis inhibition rate as the ordinate model.

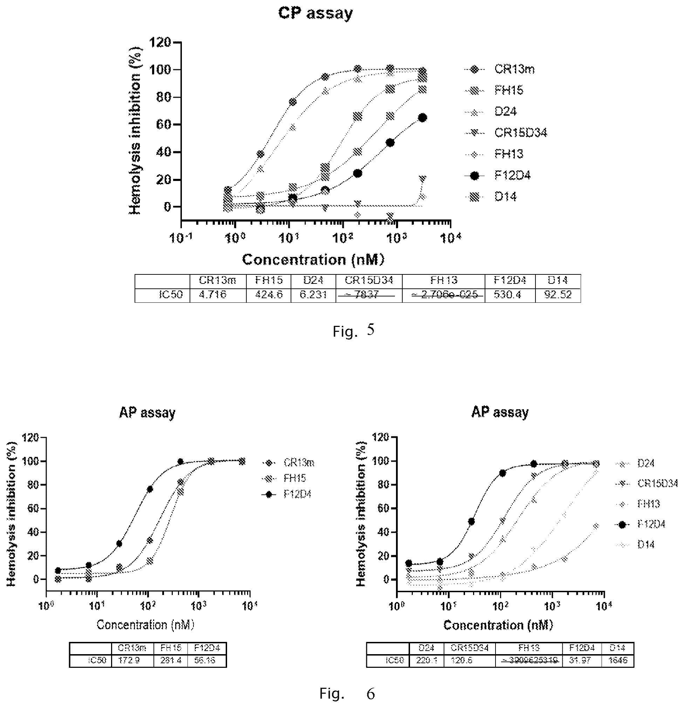

The experimental test results of inhibitory activity toward CP hemolysis showed that the hybrid protein of CFH and DAF, F12D4, had weaker CP-inhibitory activity, which was far weaker than that of CR1 activity-enhancing fragment CR13m and DAF fragment D24, and significantly weaker than that of CFH fragment FH15 and DAF fragment D14. The hybrid protein of CR1 and DAF, CR15D34, only has weak CP-inhibitory activity, while CFH fragment FH13 had little activity (as shown in Figure 5), indicating that both hybrid proteins obtained through domain hybridization had weaker CP-inhibitory activity.

4.2 Detection of inhibitory activity on Alternative Pathway of complement (AP)

In this experiment, the degree of inhibition of rabbit erythrocyte hemolysis caused by normal human serum complement activity was measured by testing the protein to be tested at different concentrations, and the inhibitory activity of each protein on complement AP was analyzed and compared.

The experimental process was briefly described as follows:

(1) Gradient dilution of proteins: The protein was formulated into a 28000nM solution with GVBMG buffer (Gelatin Flora Buffer comprising Mg2+ and EGTA, Cat. No.: 25-02090, TIANDZ), followed by 4-fold gradient dilution, with a total of 7 gradients;

(2) Dilution of serum: The normal human serum NHS (Shanghai Schbio Co., Ltd.) was taken out of the -70°C refrigerator, followed by naturally thawing at 4°C and the serum was diluted to 60% with GVBMG;

(3) Preparation of rabbit red blood cells: rabbit red blood cells (Nanjing SenBeiJia Biological Technology Co., Ltd.) were washed with GVBMG until the supernatant was transparent, and then resuspended with GVBMG;

(4) Incubation: 25 μL each of the diluted protein and serum was mixed in a 96-well plate, then 50 μL of the red blood cell suspension obtained in step (3) was added, resulting in an initial final concentration of the protein of 7000 nM and a final concentration of the serum of 15%, the mixture was blended thoroughly and placed in a 37°C constant temperature incubator for 1 hour, with a negative control (comprising only serum) and a positive control (comprising only serum and red blood cells);

(5) Termination: After the incubation, 100 μL of 10 mM EDTA-GVB buffer (GVBE) was added to each reaction well to terminate the reaction;

(6) Reading value: The 96-well plate was centrifuged at 3000 rpm for 5 minutes. 100 μL of the supernatant was added into a new 96-well flat-bottom plate. The absorbance value at OD405nm was measured in a multi-function plate reader, and the data were saved;

(7) Data processing: the obtained OD405nm reading value was put into the following formula to calculate the red blood cell hemolysis inhibition rate:

Hemolysis inhibition rate (%) = (positive control reading value - experimental group reading value) / (positive control reading value - negative control reading value) * 100%

Then the IC50 value of the measured inhibitory protein was calculated by plotting a four-parameter fit curve, with the final protein concentration as the abscissa and the hemolysis inhibition rate as the ordinate model. The results were shown in Figure 6.

The experimental test results of inhibitory activity toward AP hemolysis showed that the hybrid protein of CFH and DAF, F12D4, had potent AP-inhibitory activity, which was significantly better than that of CFH fragment FH15 and CR1 activity-enhancing fragment CR13m, and also significantly superior to that of DAF fragments D24, D14, and CFH fragment FH13 that only has weak AP-inhibitory activity. Another hybrid protein of CR1 and DAF, CR15D34, also had good AP-inhibitory activity, which was superior to that of DAF fragments D24, D14 and CFH fragment FH13, and approximately equivalent to that of CFH fragment FH15 and CR1 activity-enhancing fragment CR13m (as shown in Figure 6), indicating that both hybrid proteins obtained through domain hybridization had biased inhibitory activity toward AP, with F12D4 having greater AP inhibitory activity.

**Sequence listing**

| SEQ ID NO:1 | MTVARPSVPAALPLLGELPRLLLLVLLCLPAVWGDCGLPPDVPNAQPALEGRTSFPEDTVITYK<br>CEESFVKIPGEKDSVICLKGSQWSDIEEFCNRSCEVPTRLNSASLKQPYITQNYFPVGTVVEYE<br>CRPGYRREPSLSPKLTCLQNLKWSTAVEFCKKKSCPNPGEIRNGQIDVPGGILFGATISFSCNTG<br>YKLFGSTSSFCLISGSSVQWSDPLPECREIYCPAPPQIDNGIIQGERDHYGYRQSVTYACNKGF<br>TMIGEHSIYCTVNNDEGEWSGPPPECRGKSLTSKVPPTVQKPTTVNVPTTEVSPTSQKTTTKT<br>TTPNAQATRSTPVSRTTKHFHETTPNKGSGTTSGTTRLLSGHTCFTLTGLLGTLVTMGLLT |
|---|---|
| SEQ ID NO:2 | ATGACCGTCGCGCGGCCGAGCGTGCCCGCGGCGCTGCCCCTCCTCGGGGAGCTGCCCCG<br>GCTGCTGCTGCTGGTGCTGTTGTGCCTGCCGGCCGTGTGGGGTGACTGTGGCCTTCCCCC<br>AGATGTACCTAATGCCCAGCCAGCTTTGGAAGGCCGTACAAGTTTTCCCGAGGATACTGTA<br>ATAACGTACAAATGTGAAGAAAGCTTTGTGAAAATTCCTGGCGAGAAGGACTCAGTGATC<br>TGCCTTAAGGGCAGTCAATGGTCAGATATTGAAGAGTTCTGCAATCGTAGCTGCGAGGTG<br>CCAACAAGGCTAAATTCTGCATCCCTCAAACAGCCTTATATCACTCAGAATTATTTTCCAGT<br>CGGTACTGTTGTGGAATATGAGTGCCGTCCAGGTTACAGAAGAGAACCTTCTCTATCACCA<br>AAACTAACTTGCCTTCAGAATTTAAAATGGTCCACAGCAGTCGAATTTTGTAAAAAGAAAT<br>CATGCCCTAATCCGGGAGAAATACGAAATGGTCAGATTGATGTACCAGGTGGCATATTATTT<br>GGTGCAACCATCTCCTTCTCATGTAACACAGGGTACAAATTATTTGGCTCGACTTCTAGTTT<br>TTGTCTTATTTCAGGCAGCTCTGTCCAGTGGAGTGACCCGTTGCCAGAGTGCAGAGAAAT<br>TTATTGTCCAGCACCACCACAAATTGACAATGGAATAATTCAAGGGGAACGTGACCATTAT<br>GGATATAGACAGTCTGTAACGTATGCATGTAATAAAGGATTCACCATGATTGGAGAGCACT<br>CTATTTATTGTACTGTGAATAATGATGAAGGAGAGTGGAGTGGCCCACCACCTGAATGCAG<br>AGGAAAATCTCTAACTTCCAAGGTCCCACCAACAGTTCAGAAACCTACCACAGTAAATGT<br>TCCAACTACAGAAGTCTCACCAACTTCTCAGAAAACCACCACAAAAACCACCACACCAA<br>ATGCTCAAGCAACACGGAGTACACCTGTTTCCAGGACAACCAAGCATTTTCATGAAACAA<br>CCCCAAATAAAGGAAGTGGAACCACTTCAGGTACTACCCGTCTTCTATCTGGGCACACGT<br>GTTTCACGTTGACAGGTTTGCTTGGGACGCTAGTAACCATGGGCTTGCTGACTTAG |

(continued)

| SEQ ID NO:3 | MRLLAKIICLMLWAICVAEDCNELPPRRNTEILTGSWSDQTYPEGTQAIYKCRPGYRSLGNVI MVCRKGEWVALNPLRKCQKRPCGHPGDTPFGTFTLTGGNVFEYGVKAVYTCNEGYQLLGEI NYRECDTDGWTNDIPICEVVKCLPVTAPENGKIVSSAMEPDREYHFGQAVRFVCNSGYKIEG DEEMHCSDDGFWSKEKPKCVEISCKSPDVINGSPISQKIIYKENERFQYKCNMGYEYSERGDA VCTESGWRPLPSCEEKSCDNPYIPNGDYSPLRIKHRTGDEITYQCRNGFYPATRGNTAKCTST GWIPAPRCTLKPCDYPDIKHGGLYHENMRRPYFPVAVGKYYSYYCDEHFETPSGSYWDHIHC TQDGWSPAVPCLRKCYFPYLENGYNQNYGRKFVQGKSIDVACHPGYALPKAQTTVTCMENG WSPTPRCIRVKTCSKSSIDIENGFISESQYTYALKEKAKYQCKLGYVTADGETSGSITCGKDG WSAQPTCIKSCDIPVFMNARTKNDFTWFKLNDTLDYECHDGYESNTGSTTGSIVCGYNGWS DLPICYERECELPKIDVHLVPDRKKDQYKVGEVLKFSCKPGFTIVGPNSVQCYHFGLSPDLPIC KEQVQSCGPPPELLNGNVKEKTKEEYGHSEVVEYYCNPRFLMKGPNKIQCVDGEWTTLPVC IVEESTCGDIPELEHGWAQLSSPPYYYGDSVEFNCSESFTMIGHRSITCIHGVWTQLPQCVAID KLKKCKSSNLIILEEHLKNKKEFDHNSNIRYRCRGKEGWIHTVCINGRWDPEVNCSMAQIQL CPPPPQIPNSHNMTTTLNYRDGEKVSVLCQENYLIQEGEEITCKDGRWQSIPLCVEKIPCSQPP QIEHGTINSSRSSQESYAHGTKLSYTCEGGFRISEENETTCYMGKWSSPPQCEGLPCKSPPEISH GVVAHMSDSYQYGEEVTYKCFEGFGIDGPAIAKCLGEKWSHPPSCIKTDCLSLPSFENAIPMG EKKDVYKAGEQVTYTCATYYKMDGASNVTCINSRWTGRPTCRDTSCVNPPTVQNAYIVSRQ MSKYPSGERVRYQCRSPYEMFGDEEVMCLNGNWTEPPQCKDSTGKCGPPPPIDNGDITSFPL SVYAPASSVEYQCQNLYQLEGNKRITCRNGQWSEPPKCLHPCVISREIMENYNIALRWTAKQ KLYSRTGESVEFVCKRGYRLSSRSHTLRTTCWDGKLEYPTCAKR |
|---|---|

(continued)

| SEQ ID NO:4 | ATGAGACTTCTAGCAAAGATTATTTGCCTTATGTTATGGGCTATTTGTGTAGCAGAAGATTG CAATGAACTTCCTCCAAGAAGAAATACAGAAATTCTGACAGGTTCCTGGTCTGACCAAAC ATATCCAGAAGGCACCCAGGCTATCTATAAATGCCGCCCTGGATATAGATCTCTTGGAAATG TAATAATGGTATGCAGGAAGGGAGAATGGGTTGCTCTTAATCCATTAAGGAAATGTCAGAA AAGGCCCTGTGGACATCCTGGAGATACTCCTTTTGGTACTTTTACCCTTACAGGAGGAAAT GTGTTTGAATATGGTGTAAAAGCTGTGTATACATGTAATGAGGGGTATCAATTGCTAGGTGA GATTAATTACCGTGAATGTGACACAGATGGATGGACCAATGATATTCCTATATGTGAAGTTG TGAAGTGTTTACCAGTGACAGCACCAGAGAATGGAAAAATTGTCAGTAGTGCAATGGAAC CAGATCGGGAATACCATTTTGGACAAGCAGTACGGTTTGTATGTAACTCAGGCTACAAGAT TGAAGGAGATGAAGAAATGCATTGTTCAGACGATGGTTTTTGGAGTAAAGAGAAACCAA AGTGTGTGGAAATTTCATGCAAATCCCCAGATGTTATAAATGGATCTCCTATATCTCAGAAG ATTATTTATAAGGAGAATGAACGATTTCAATATAAATGTAACATGGGTTATGAATACAGTGA AAGAGGAGATGCTGTATGCACTGAATCTGGATGGCGTCCGTTGCCTTCATGTGAAGAAAA ATCATGTGATAATCCTTATATTCCAAATGGTGACTACTCACCTTTAAGGATTAAACACAGAA CTGGAGATGAAATCACGTACCAGTGTAGAAATGGTTTTTATCCTGCAACCCGGGGAAATAC AGCAAAATGCACAAGTACTGGCTGGATACCTGCTCCGAGATGTACCTTGAAACCTTGTGAT TATCCAGACATTAAACATGGAGGTCTATATCATGAGAATATGCGTAGACCATACTTTCCAGT AGCTGTAGGAAAATATTACTCCTATTACTGTGATGAACATTTTGAGACTCCGTCAGGAAGT TACTGGGATCACATTCATTGCACACAAGATGGATGGTCGCCAGCAGTACCATGCCTCAGAA AATGTTATTTTCCTTATTTGGAAAATGGATATAATCAAAATCATGGAAGAAAGTTTGTACAG GGTAAATCTATAGACGTTGCCTGCCATCCTGGCTACGCTCTTCCAAAAGCGCAGACCACAG TTACATGTATGGAGAATGGCTGGTCTCCTACTCCCAGATGCATCCGTGTCAAAACATGTTC CAAATCAAGTATAGATATTGAGAATGGGTTTATTTCTGAATCTCAGTATACATATGCCTTAAA AGAAAAAGCGAAATATCAATGCAAACTAGGATATGTAACAGCAGATGGTGAAACATCAGG ATCAATTACATGTGGGAAAGATGGATGGTCAGCTCAACCCACGTGCATTAAATCTTGTGAT ATCCCAGTATTTATGAATGCCAGAACTAAAAATGACTTCACATGGTTTAAGCTGAATGACA CATTGGACTATGAATGCCATGATGGTTATGAAAGCAATACTGGAAGCACCACTGGTTCCAT AGTGTGTGGTTACAATGGTTGGTCTGATTTACCCATATGTTATGAAAGAGAATGCGAACTT CCTAAAATAGATGTACACTTAGTTCCTGATCGCAAGAAAGACCAGTATAAAGTTGGAGAG GTGTTGAAATTCTCCTGCAAACCAGGATTTACAATAGTTGGACCTAATTCCGTTCAGTGCT ACCACTTTGGATTGTCTCCTGACCTCCCAATATGTAAAGAGCAAGTACAATCATGTGGTCC ACCTCCTGAACTCCTCAATGGGAATGTTAAGGAAAAAACGAAAGAAGAATATGGACACA GTGAAGTGGTGGAATATTATTGCAATCCTAGATTTCTAATGAAGGGACCTAATAAAATTCAA TGTGTTGATGGAGAGTGGACAACTTTACCAGTGTGTATTGTGGAGGAGAGTACCTGTGGA GATATACCTGAACTTGAACATGGCTGGGCCCAGCTTTCTTCCCCTCCTTATTACTATGGAGA TTCAGTGGAATTCAATTGCTCAGAATCATTTACAATGATTGGACACAGATCAATTACGTGTA TTCATGGAGTATGGACCCAACTTCCCCAGTGTGTGGCAATAGATAAACTTAAGAAGTGCA AATCATCAAATTTAATTATACTTGAGGAACATTTAAAAAACAAGAAGGAATTCGATCATAAT TCTAACATAAGGTACAGATGTAGAGGAAAAGAAGGATGGATACACACAGTCTGCATAAAT GGAAGATGGGATCCAGAAGTGAACTGCTCAATGGCACAAATACAATTATGCCCACCTCCA |

(continued)

| | |
|---|---|
| | CCTCAGATTCCCAATTCTCACAATATGACAACCACACTGAATTATCGGGATGGAGAAAAG<br>TATCTGTTCTTTGCCAAGAAAATTATCTAATTCAGGAAGGAGAAGAAATTACATGCAAGA<br>TGGAAGATGGCAGTCAATACCACTCTGTGTTGAAAAAATTCCATGTTCACAACCACCTCA<br>GATAGAACACGGAACCATTAATTCATCCAGGTCTTCACAAGAAAGTTATGCACATGGGACT<br>AAATTGAGTTATACTTGTGAGGGTGGTTTCAGGATATCTGAAGAAATGAAACAACATGCT<br>ACATGGGAAAATGGAGTTCTCCACCTCAGTGTGAAGGCCTTCCTTGTAAATCTCCACCTG<br>AGATTTCTCATGGTGTTGTAGCTCACATGTCAGACAGTTATCAGTATGGAGAAGAAGTTAC<br>GTACAAATGTTTTGAAGGTTTTGGAATTGATGGGCCTGCAATTGCAAATGCTTAGGAGAA<br>AAATGGTCTCACCCTCCATCATGCATAAAAACAGATTGTCTCAGTTTACCTAGCTTTGAAA<br>ATGCCATACCCATGGGAGAGAAGAAGGATGTGTATAAGGCGGGTGAGCAAGTGACTTACA<br>CTTGTGCAACATATTACAAAATGGATGGAGCCAGTAATGTAACATGCATTAATAGCAGATG<br>GACAGGAAGGCCAACATGCAGAGACACCTCCTGTGTGAATCCGCCCACAGTACAAAATG<br>CTTATATAGTGTCGAGACAGATGAGTAAATATCCATCTGGTGAGAGAGTACGTTATCAATGT<br>AGGAGCCCTTATGAAATGTTTGGGGATGAAGAAGTGATGTGTTTAAATGGAAACTGGACG<br>GAACCACCTCAATGCAAAGATTCTACAGGAAAATGTGGGCCCCCTCCACCTATTGACAAT<br>GGGGACATTACTTCATTCCCGTTGTCAGTATATGCTCCAGCTTCATCAGTTGAGTACCAATG<br>CCAGAACTTGTATCAACTTGAGGGTAACAAGCGAATAACATGTAGAAATGGACAATGGTC<br>AGAACCACCAAAATGCTTACATCCGTGTGTAATATCCCGAGAAATTATGGAAAATTATAAC<br>ATAGCATTAAGGTGGACAGCCAAACAGAAGCTTTATTCGAGAACAGGTGAATCAGTTGAA<br>TTTGTGTGTAAACGGGGATATCGTCTTTCATCACGTTCTCACACATTGCGAACAACATGTT<br>GGGATGGGAAACTGGAGTATCCAACTTGTGCAAAAAGATAG |
| SEQ<br>ID<br>NO:5 | MRLLAKIICLMLWAICVAEDCNELPPRRNTEILTGSWSDQTYPEGTQAIYKCRPGYRSLGNVI<br>MVCRKGEWVALNPLRKCQKRPCGHPGDTPFGTFTLTGGNVFEYGVKAVYTCNEGYQLLGEI<br>NYRECDTDGWTNDIPICEVVKCLPVTAPENGKIVSSAMEPDREYHFGQAVRFVCNSGYKIEG<br>DEEMHCSDDGFWSKEKPKCVEISCKSPDVINGSPISQKIIYKENERFQYKCNMGYEYSERGDA<br>VCTESGWRPLPSCEEKSCDNPYIPNGDYSPLRIKHRTGDEITYQCRNGFYPATRGNTAKCTST<br>GWIPAPRCTLKPCDYPDIKHGGLYHENMRRPYFPVAVGKYYSYYCDEHFETPSGSYWDHIHC<br>TQDGWSPAVPCLRKCYFPYLENGYNQNYGRKFVQGKSIDVACHPGYALPKAQTTVTCMENG<br>WSPTPRCIRVSFTL |

(continued)

| SEQ ID NO:6 | ATGAGACTTCTAGCAAAGATTATTTGCCTTATGTTATGGGCTATTTGTGTAGCAGAAGATTG CAATGAACTTCCTCCAAGAAGAAATACAGAAATTCTGACAGGTTCCTGGTCTGACCAAAC ATATCCAGAAGGCACCCAGGCTATCTATAAATGCCGCCCTGGATATAGATCTCTTGGAAATG TAATAATGGTATGCAGGAAGGGAGAATGGGTTGCTCTTAATCCATTAAGGAAATGTCAGAA AAGGCCCTGTGGACATCCTGGAGATACTCCTTTTGGTACTTTTACCCTTACAGGAGGAAAT GTGTTTGAATATGGTGTAAAAGCTGTGTATACATGTAATGAGGGGTATCAATTGCTAGGTGA GATTAATTACCGTGAATGTGACACAGATGGATGGACCAATGATATTCCTATATGTGAAGTTG TGAAGTGTTTACCAGTGACAGCACCAGAGAATGGAAAAATTGTCAGTAGTGCAATGGAAC CAGATCGGGAATACCATTTTGGACAAGCAGTACGGTTTGTATGTAACTCAGGCTACAAGAT TGAAGGAGATGAAGAAATGCATTGTTCAGACGATGGTTTTTGGAGTAAAGAGAAACCAA AGTGTGTGGAAATTTCATGCAAATCCCCAGATGTTATAAATGGATCTCCTATATCTCAGAAG ATTATTTATAAGGAGAATGAACGATTTCAATATAAATGTAACATGGGTTATGAATACAGTGA AAGAGGAGATGCTGTATGCACTGAATCTGGATGGCGTCCGTTGCCTTCATGTGAAGAAAA ATCATGTGATAATCCTTATATTCCAAATGGTGACTACTCACCTTTAAGGATTAAACACAGAA CTGGAGATGAAATCACGTACCAGTGTAGAAATGGTTTTTATCCTGCAACCCGGGGAAATAC AGCAAAATGCACAAGTACTGGCTGGATACCTGCTCCGAGATGTACCTTGAAACCTTGTGAT TATCCAGACATTAAACATGGAGGTCTATATCATGAGAATATGCGTAGACCATACTTTCCAGT AGCTGTAGGAAAATATTACTCCTATTACTGTGATGAACATTTTGAGACTCCGTCAGGAAGT TACTGGGATCACATTCATTGCACACAAGATGGATGGTCGCCAGCAGTACCATGCCTCAGAA AATGTTATTTTCCTTATTTGGAAAATGGATATAATCAAAATCATGGAAGAAAGTTTGTACAG GGTAAATCTATAGACGTTGCCTGCCATCCTGGCTACGCTCTTCCAAAAGCGCAGACCACAG TTACATGTATGGAGAATGGCTGGTCTCCTACTCCCAGATGCATCCGTGTCAGCTTTACCCTC TGA |

| SEQ ID NO:7 | MGASSPRSPEPVGPPAPGLPFCCGGSLLAVVVLLALPVAWGQCNAPEWLPFARPTNLTDEFEF PIGTYLNYECRPGYSGRPFSIICLKNSVWTGAKDRCRRKSCRNPPDPVNGMVHVIKGIQFGSQ IKYSCTKGYRLIGSSSATCIISGDTVIWDNETPICDRIPCGLPPTITNGDFISTNRENFHYGSVVT YRCNPGSGGRKVFELVGEPSIYCTSNDDQVGIWSGPAPQCIIPNKCTPPNVENGILVSDNRSLF SLNEVVEFRCQPGFVMKGPRRVKCQALNKWEPELPSCSRVCQPPPDVLHAERTQRDKDNFSP GQEVFYSCEPGYDLRGAASMRCTPQGDWSPAAPTCEVKSCDDFMGQLLNGRVLFPVNLQLG AKVDFVCDEGFQLKGSSASYCVLAGMESLWNSSVPVCEQIFCPSPPVIPNGRHTGKPLEVFPF GKTVNYTCDPHPDRGTSFDLIGESTIRCTSDPQGNGVWSSPAPRCGILGHCQAPDHFLFAKLK TQTNASDFPIGTSLKYECRPEYYGRPFSITCLDNLVWSSPKDVCKRKSCKTPPDPVNGMVHVI TDIQVGSRINYSCTTGHRLIGHSSAECILSGNAAHWSTKPPICQRIPCGLPPTIANGDFISTNRE NFHYGSVVTYRCNPGSGGRKVFELVGEPSIYCTSNDDQVGIWSGPAPQCIIPNKCTPPNVENGI LVSDNRSLFSLNEVVEFRCQPGFVMKGPRRVKCQALNKWEPELPSCSRVCQPPPDVLHAERT QRDKDNFSPGQEVFYSCEPGYDLRGAASMRCTPQGDWSPAAPTCEVKSCDDFMGQLLNGR VLFPVNLQLGAKVDFVCDEGFQLKGSSASYCVLAGMESLWNSSVPVCEQIFCPSPPVIPNGRH TGKPLEVFPFGKAVNYTCDPHPDRGTSFDLIGESTIRCTSDPQGNGVWSSPAPRCGILGHCQAP DHFLFAKLKTQTNASDFPIGTSLKYECRPEYYGRPFSITCLDNLVWSSPKDVCKRKSCKTPPD PVNGMVHVITDIQVGSRINYSCTTGHRLIGHSSAECILSGNTAHWSTKPPICQRIPCGLPPTIAN GDFISTNRENFHYGSVVTYRCNLGSRGRKVFELVGEPSIYCTSNDDQVGIWSGPAPQCIIPNKC TPPNVENGILVSDNRSLFSLNEVVEFRCQPGFVMKGPRRVKCQALNKWEPELPSCSRVCQPPP EILHGEHTPSHQDNFSPGQEVFYSCEPGYDLRGAASLHCTPQGDWSPEAPRCAVKSCDDFLG QLPHGRVLFPLNLQLGAKVSFVCDEGFRLKGSSVSHCVLVGMRSLWNNSVPVCEHIFCPNPP AILNGRHTGTPSGDIPYGKEISYTCDPHPDRGMTFNLIGESTIRCTSDPHGNGVWSSPAPRCEL SVRAGHCKTPEQFPFASPTIPINDFEFPVGTSLNYECRPGYFGKMFSISCLENLVWSSVEDNCR RKSCGPPPEPFNGMVHINTDTQFGSTVNYSCNEGFRLIGSPSTTCLVSGNNVTWDKKAPICEII SCEPPPTISNGDFYSNNRTSFHNGTVVTYQCHTGPDGEQLFELVGERSIYCTSKDDQVGVWSS PPPRCISTNKCTAPEVENAIRVPGNRSFFTLTEIIRFRCQPGFVMVGSHTVQCQTNGRWGPKLP HCSRVCQPPPEILHGEHTLSHQDNFSPGQEVFYSCEPSYDLRGAASLHCTPQGDWSPEAPRCT VKSCDDFLGQLPHGRVLLPLNLQLGAKVSFVCDEGFRLKGRSASHCVLAGMKALWNSSVPV CEQIFCPNPPAILNGRHTGTPFGDIPYGKEISYACDTHPDRGMTFNLIGESSIRCTSDPQGNGV WSSPAPRCELSVPAACPHPPKIQNGHYIGGHVSLYLPGMTISYICDPGYLLVGKGFIFCTDQGI WSQLDHYCKEVNCSFPLFMNGISKELEMKKVYHYGDYVTLKCEDGYTLEGSPWSQCQADD RWDPPLAKCTSRTHDALIVGTLSGTIFFILLIIFLSWIILKHRKGNNAHENPKEVAIHLHSQGGS SVHPRTLQTNEENSRVLP |
| SEQ ID NO:8 | ATGGGGGCCTCTTCTCCAAGAAGCCCGGAGCCTGTCGGGCCGCCGGCGCCCGGTCTCCCC TTCTGCTGCGGAGGATCCCTGCTGGCGGTTGTGGTGCTGCTTGCGCTGCCGGTGGCCTGG GGTCAATGCAATGCCCCAGAATGGCTTCCATTTGCCAGGCCTACCAACCTAACTGATGAAT TTGAGTTTCCCATTGGGACATATCTGAACTATGAATGCCGCCCTGGTTATTCCGGAAGACC GTTTTCTATCATCTGCCTAAAAAACTCAGTCTGGACTGGTGCTAAGGACAGGTGCAGACG |

```
TAAATCATGTCGTAATCCTCCAGATCCTGTGAATGGCATGGTGCATGTGATCAAAGGCATC
CAGTTCGGATCCCAAATTAAATATTCTTGTACTAAAGGATACCGACTCATTGGTTCCTCGTC
TGCCACATGCATCATCTCAGGTGATACTGTCATTTGGGATAATGAAACACCTATTTGTGACA
GAATTCCTTGTGGGCTACCCCCCACCATCACCAATGGAGATTTCATTAGCACCAACAGAGA
GAATTTTCACTATGGATCAGTGGTGACCTACCGCTGCAATCCTGGAAGCGGAGGGAGAAA
GGTGTTTGAGCTTGTGGGTGAGCCCTCCATATACTGCACCAGCAATGACGATCAAGTGGG
CATCTGGAGCGGCCCCGCCCCTCAGTGCATTATACCTAACAAATGCACGCCTCCAAATGTG
GAAAATGGAATATTGGTATCTGACAACAGAAGCTTATTTTCCTTAAATGAAGTTGTGGAGT
TTAGGTGTCAGCCTGGCTTTGTCATGAAAGGACCCCGCCGTGTGAAGTGCCAGGCCCTGA
ACAAATGGGAGCCGGAGCTACCAAGCTGCTCCAGGGTATGTCAGCCACCTCCAGATGTCC
TGCATGCTGAGCGTACCCAAAGGGACAAGGACAACTTTTCACCTGGGCAGGAAGTGTTCT
ACAGCTGTGAGCCCGGCTACGACCTCAGAGGGGCTGCGTCTATGCGCTGCACACCCCAGG
GAGACTGGAGCCCTGCAGCCCCCACATGTGAAGTGAAATCCTGTGATGACTTCATGGGCC
AACTTCTTAATGGCCGTGTGCTATTTCCAGTAAATCTCCAGCTTGGAGCAAAAGTGGATTT
TGTTTGTGATGAAGGATTTCAATTAAAAGGCAGCTCTGCTAGTTACTGTGTCTTGGCTGGA
ATGGAAAGCCTTTGGAATAGCAGTGTTCCAGTGTGTGAACAAATCTTTTGTCCAAGTCCTC
CAGTTATTCCTAATGGGAGACACACAGGAAAACCTCTGGAAGTCTTTCCCTTTGGGAAAA
CAGTAAATTACACATGCGACCCCCACCCAGACAGAGGGACGAGCTTCGACCTCATTGGAG
AGAGCACCATCCGCTGCACAAGTGACCCTCAAGGGAATGGGGTTTGGAGCAGCCCTGCC
CCTCGCTGTGGAATTCTGGGTCACTGTCAAGCCCCAGATCATTTTCTGTTTGCCAAGTTGA
AAACCCAAACCAATGCATCTGACTTTCCCATTGGGACATCTTTAAAGTACGAATGCCGTCC
TGAGTACTACGGGAGGCCATTCTCTATCACATGTCTAGATAACCTGGTCTGGTCAAGTCCC
AAAGATGTCTGTAAACGTAAATCATGTAAAACTCCTCCAGATCCAGTGAATGGCATGGTGC
ATGTGATCACAGACATCCAGGTTGGATCCAGAATCAACTATTCTTGTACTACAGGGCACCG
ACTCATTGGTCACTCATCTGCTGAATGTATCCTCTCGGGCAATGCTGCCCATTGGAGCACG
AAGCCGCCAATTTGTCAACGAATTCCTTGTGGGCTACCCCCCACCATCGCCAATGGAGATT
TCATTAGCACCAACAGAGAGAATTTTCACTATGGATCAGTGGTGACCTACCGCTGCAATCC
TGGAAGCGGAGGGAGAAAGGTGTTTGAGCTTGTGGGTGAGCCCTCCATATACTGCACCAG
CAATGACGATCAAGTGGGCATCTGGAGCGGCCCGGCCCCTCAGTGCATTATACCTAACAA
ATGCACGCCTCCAAATGTGGAAAATGGAATATTGGTATCTGACAACAGAAGCTTATTTTCC
TTAAATGAAGTTGTGGAGTTTAGGTGTCAGCCTGGCTTTGTCATGAAAGGACCCCGCCGT
GTGAAGTGCCAGGCCCTGAACAAATGGGAGCCGGAGCTACCAAGCTGCTCCAGGGTATG
TCAGCCACCTCCAGATGTCCTGCATGCTGAGCGTACCCAAAGGGACAAGGACAACTTTTC
ACCCGGGCAGGAAGTGTTCTACAGCTGTGAGCCCGGCTATGACCTCAGAGGGGCTGCGTC
TATGCGCTGCACACCCCAGGGAGACTGGAGCCCTGCAGCCCCCACATGTGAAGTGAAATC
CTGTGATGACTTCATGGGCCAACTTCTTAATGGCCGTGTGCTATTTCCAGTAAATCTCCAGC
TTGGAGCAAAAGTGGATTTTGTTTGTGATGAAGGATTTCAATTAAAAGGCAGCTCTGCTAG
TTATTGTGTCTTGGCTGGAATGGAAAGCCTTTGGAATAGCAGTGTTCCAGTGTGTGAACAA
ATCTTTTGTCCAAGTCCTCCAGTTATTCCTAATGGGAGACACACAGGAAAACCTCTGGAA
GTCTTTCCCTTTGGAAAAGCAGTAAATTACACATGCGACCCCCACCCAGACAGAGGGACG
AGCTTCGACCTCATTGGAGAGAGCACCATCCGCTGCACAAGTGACCCTCAAGGGAATGG
GGTTTGGAGCAGCCCTGCCCCTCGCTGTGGAATTCTGGGTCACTGTCAAGCCCCAGATCA
TTTTCTGTTTGCCAAGTTGAAAACCCAAACCAATGCATCTGACTTTCCCATTGGGACATCT
TTAAAGTACGAATGCCGTCCTGAGTACTACGGGAGGCCATTCTCTATCACATGTCTAGATA
ACCTGGTCTGGTCAAGTCCCAAAGATGTCTGTAAACGTAAATCATGTAAAACTCCTCCAG
```

ATCCAGTGAATGGCATGGTGCATGTGATCACAGACATCCAGGTTGGATCCAGAATCAACTA
TTCTTGTACTACAGGGCACCGACTCATTGGTCACTCATCTGCTGAATGTATCCTCTCAGGC
AATACTGCCCATTGGAGCACGAAGCCGCCAATTTGTCAACGAATTCCTTGTGGGCTACCCC
CAACCATCGCCAATGGAGATTTCATTAGCACCAACAGAGAGAATTTTCACTATGGATCAGT
GGTGACCTACCGCTGCAATCTTGGAAGCAGAGGGAGAAAGGTGTTTGAGCTTGTGGGTG
AGCCCTCCATATACTGCACCAGCAATGACGATCAAGTGGGCATCTGGAGCGGCCCCGCCC
CTCAGTGCATTATACCTAACAAATGCACGCCTCCAAATGTGGAAAATGGAATATTGGTATCT
GACAACAGAAGCTTATTTTCCTTAAATGAAGTTGTGGAGTTTAGGTGTCAGCCTGGCTTTG
TCATGAAAGGACCCCGCCGTGTGAAGTGCCAGGCCCTGAACAAATGGGAGCCAGAGTTA
CCAAGCTGCTCCAGGGTGTGTCAGCCGCCTCCAGAAATCCTGCATGGTGAGCATACCCCA
AGCCATCAGGACAACTTTTCACCTGGGCAGGAAGTGTTCTACAGCTGTGAGCCTGGCTAT
GACCTCAGAGGGGCTGCGTCTCTGCACTGCACACCCCAGGGAGACTGGAGCCCTGAAGC
CCCGAGATGTGCAGTGAAATCCTGTGATGACTTCTTGGGTCAACTCCCTCATGGCCGTGTG
CTATTTCCACTTAATCTCCAGCTTGGGGCAAAGGTGTCCTTTGTCTGTGATGAAGGGTTTC
GCTTAAAGGGCAGTTCCGTTAGTCATTGTGTCTTGGTTGGAATGAGAAGCCTTTGGAATAA
CAGTGTTCCTGTGTGTGAACATATCTTTTGTCCAAATCCTCCAGCTATCCTTAATGGGAGAC
ACACAGGAACTCCCTCTGGAGATATTCCCTATGGAAAAGAAATATCTTACACATGTGACCC
CCACCCAGACAGAGGGATGACCTTCAACCTCATTGGGGAGAGCACCATCCGCTGCACAA
GTGACCCTCATGGGAATGGGGTTTGGAGCAGCCCTGCCCCTCGCTGTGAACTTTCTGTTC
GTGCTGGTCACTGTAAAACCCCAGAGCAGTTTCCATTTGCCAGTCCTACGATCCCAATTAA
TGACTTTGAGTTTCCAGTCGGGACATCTTTGAATTATGAATGCCGTCCTGGGTATTTTGGG
AAAATGTTCTCTATCTCCTGCCTAGAAAACTTGGTCTGGTCAAGTGTTGAAGACAACTGTA
GACGAAAATCATGTGGACCTCCACCAGAACCCTTCAATGGAATGGTGCATATAAACACAG
ATACACAGTTTGGATCAACAGTTAATTATTCTTGTAATGAAGGGTTTCGACTCATTGGTTCC
CCATCTACTACTTGTCTCGTCTCAGGCAATAATGTCACATGGGATAAGAAGGCACCTATTTG
TGAGATCATATCTTGTGAGCCACCTCCAACCATATCCAATGGAGACTTCTACAGCAACAAT
AGAACATCTTTTCACAATGGAACGGTGGTAACTTACCAGTGCCACACTGGACCAGATGGA
GAACAGCTGTTTGAGCTTGTGGGAGAACGGTCAATATATTGCACCAGCAAAGATGATCAA
GTTGGTGTTTGGAGCAGCCCTCCCCCTCGGTGTATTTCTACTAATAAATGCACAGCTCCAG
AAGTTGAAAATGCAATTAGAGTACCAGGAAACAGGAGTTTCTTTACCCTCACTGAGATCA
TCAGATTTAGATGTCAGCCCGGGTTTGTCATGGTAGGGTCCCACACTGTGCAGTGCCAGA
CCAATGGCAGATGGGGGCCCAAGCTGCCACACTGCTCCAGGGTGTGTCAGCCGCCTCCA
GAAATCCTGCATGGTGAGCATACCCTAAGCCATCAGGACAACTTTTCACCTGGGCAGGAA
GTGTTCTACAGCTGTGAGCCCAGCTATGACCTCAGAGGGGCTGCGTCTCTGCACTGCACG
CCCCAGGGAGACTGGAGCCCTGAAGCCCCTAGATGTACAGTGAAATCCTGTGATGACTTC
CTGGGCCAACTCCCTCATGGCCGTGTGCTACTTCCACTTAATCTCCAGCTTGGGGCAAAGG
TGTCCTTTGTTTGCGATGAAGGGTTCCGATTAAAAGGCAGGTCTGCTAGTCATTGTGTCTT
GGCTGGAATGAAAGCCCTTTGGAATAGCAGTGTTCCAGTGTGTGAACAAATCTTTTGTCC
AAATCCTCCAGCTATCCTTAATGGGAGACACACAGGAACTCCCTTTGGAGATATTCCCTAT
GGAAAAGAAATATCTTACGCATGCGACACCCACCCAGACAGAGGGATGACCTTCAACCTC
ATTGGGGAGAGCTCCATCCGCTGCACAAGTGACCCTCAAGGGAATGGGGTTTGGAGCAG
CCCTGCCCCTCGCTGTGAACTTTCTGTTCCTGCTGCCTGCCCACATCCACCCAAGATCCAA
AACGGGCATTACATTGGAGGACACGTATCTCTATATCTTCCTGGGATGACAATCAGCTACAT
TTGTGACCCCGGCTACCTGTTAGTGGGAAAGGGCTTCATTTTCTGTACAGACCAGGGAATC
TGGAGCCAATTGGATCATTATTGCAAAGAAGTAAATTGTAGCTTCCCACTGTTTATGAATG

| | |
|---|---|
| | GAATCTCGAAGGAGTTAGAAATGAAAAAAGTATATCACTATGGAGATTATGTGACTTTGAAGTGTGAAGATGGGTATACTCTGGAAGGCAGTCCCTGGAGCCAGTGCCAGGCGGATGACAGATGGGACCCTCCTCTGGCCAAATGTACCTCTCGTACACATGATGCTCTCATAGTTGGCACTTTATCTGGTACGATCTTCTTTATTTTACTCATCATTTTCCTCTCTTGGATAATTCTAAAGCACAGAAAAGGCAATAATGCACATGAAAACCCTAAAGAAGTGGCTATCCATTTACATTCTCAAGGAGGCAGCAGCGTTCATCCCCGAACTCTGCAAACAAATGAAGAAAATAGCAGGGTCCTTCCT |
| SEQ ID NO:9 | KSCPNPGEIRNGQIDVPGGILFGATISFSCNTGYKLFGSTSSFCLISGSSVQWSDPLPECRE |
| SEQ ID NO:10 | IYCPAPPQIDNGIIQGERDHYGYRQSVTYACNKGFTMIGEHSIYCTVNNDEGEWSGPPPECRG |
| SEQ ID N-O:11 | IYCPAPPQIDNGIIQGERDHYGYRQSVTYACNKGFTMIGEHSIYCTVNNDEGEWSGPPPECRGKS |
| SEQ ID N-O:12 | KSCPNPGEIRNGQIDVPGGILFGATISFSCNTGYKLFGSTSSFCLISGSSVQWSDPLPECREIYCPAPPQIDNGIIQGERDHYGYRQSVTYACNKGFTMIGEHSIYCTVNNDEGEWSGPPPECRG |
| SEQ ID N-O:13 | KSCPNPGEIRNGQIDVPGGILFGATISFSCNTGYKLFGSTSSFCLISGSSVQWSDPLPECREIYCPAPPQIDNGIIQGERDHYGYRQSVTYACNKGFTMIGEHSIYCTVNNDEGEWSGPPPECRGKS |
| SEQ ID N-O:14 | EDCNELPPRRNTEILTGSWSDQTYPEGTQAIYKCRPGYRSLGNVIMVCRKGEWVALNPLRKCQK |
| SEQ ID N-O:15 | EDCNELPPRRNTEILTGSWSDQTYPEGTQAIYKCRPGYRSLGNIIMVCRKGEWVALNPLRKCQK |
| SEQ ID N-O:16 | RPCGHPGDTPFGTFTLTGGNVFEYGVKAVYTCNEGYQLLGEINYRECDTDGWTNDIPICEV |
| SEQ ID NO:17 | RPCGHPGDTPFGTFTLTGGNVFEYGVKAVYTCN GY LLGEINYRECDTDGWTNDIPICE |
| SEQ ID NO:18 | GHCQAPDHFLFAKLKTQTNASDFPIGTSLKYECRPEYYGRPFSITCLDNLVWSSPKDVCKR |
| SEQ ID NO:19 | GHHHHHH |

| | |
|---|---|
| SEQ ID NO:20 | MGWSCIILFLVATATGVHS |
| SEQ ID N-O:21 | EDCNELPPRRNTEILTGSWSDQTYPEGTQAIYKCRPGYRSLGNVIMVCRKGEWVALNPLRKC QKRPCGHPGDTPFGTFTLTGGNVFEYGVKAVYTCN GY LLGEINYRECDTDGWTNDIPICE IYCPAPPQIDNGIIQGERDHYGYRQSVTYACNKGFTMIGEHSIYCTVNNDEGEWSGPPPECR G |
| SEQ ID N-O:22 | EDCNELPPRRNTEILTGSWSDQTYPEGTQAIYKCRPGYRSLGNIIMVCRKGEWVALNPLRKC QKRPCGHPGDTPFGTFTLTGGNVFEYGVKAVYTCN GY LLGEINYRECDTDGWTNDIPICE IYCPAPPQIDNGIIQGERDHYGYRQSVTYACNKGFTMIGEHSIYCTVNNDEGEWSGPPPECR G |
| SEQ ID N-O:23 | EDCNELPPRRNTEILTGSWSDQTYPEGTQAIYKCRPGYRSLGNVIMVCRKGEWVALNPLRKC QKRPCGHPGDTPFGTFTLTGGNVFEYGVKAVYTCN GY LLGEINYRECDTDGWTNDIPICE IYCPAPPQIDNGIIQGERDHYGYRQSVTYACNKGFTMIGEHSIYCTVNNDEGEWSGPPPECR G |
| SEQ ID N-O:24 | EDCNELPPRRNTEILTGSWSDQTYPEGTQAIYKCRPGYRSLGNIIMVCRKGEWVALNPLRKC<br><br>QKRPCGHPGDTPFGTFTLTGGNVFEYGVKAVYTCN GY LLGEINYRECDTDGWTNDIPICE IYCPAPPQIDNGIIQGERDHYGYRQSVTYACNKGFTMIGEHSIYCTVNNDEGEWSGPPPECR G |
| SEQ ID N-O:25 | EDCNELPPRRNTEILTGSWSDQTYPEGTQAIYKCRPGYRSLGNVIMVCRKGEWVALNPLRKC QKRPCGHPGDTPFGTFTLTGGNVFEYGVKAVYTCN GY LLGEINYRECDTDGWTNDIPICE IYCPAPPQIDNGIIQGERDHYGYRQSVTYACNKGFTMIGEHSIYCTVNNDEGEWSGPPPECR GKSGHHHHHH |
| SEQ ID N-O:26 | EDCNELPPRRNTEILTGSWSDQTYPEGTQAIYKCRPGYRSLGNIIMVCRKGEWVALNPLRKC QKRPCGHPGDTPFGTFTLTGGNVFEYGVKAVYTCN GY LLGEINYRECDTDGWTNDIPICE IYCPAPPQIDNGIIQGERDHYGYRQSVTYACNKGFTMIGEHSIYCTVNNDEGEWSGPPPECR GKSGHHHHHH |
| SEQ ID N-O:27 | EDCNELPPRRNTEILTGSWSDQTYPEGTQAIYKCRPGYRSLGNVIMVCRKGEWVALNPLRKC QKRPCGHPGDTPFGTFTLTGGNVFEYGVKAVYTCN GY LLGEINYRECDTDGWTNDIPICE IYCPAPPQIDNGIIQGERDHYGYRQSVTYACNKGFTMIGEHSIYCTVNNDEGEWSGPPPECR GKSGHHHHHH |
| SEQ ID N-O:28 | EDCNELPPRRNTEILTGSWSDQTYPEGTQAIYKCRPGYRSLGNIIMVCRKGEWVALNPLRKC QKRPCGHPGDTPFGTFTLTGGNVFEYGVKAVYTCN GY LLGEINYRECDTDGWTNDIPICE IYCPAPPQIDNGIIQGERDHYGYRQSVTYACNKGFTMIGEHSIYCTVNNDEGEWSGPPPECR GKSGHHHHHH |

| SEQ ID N-O:29 | GHCQAPDHFLFAKLKTQTNASDFPIGTSLKYECRPEYYGRPFSITCLDNLVWSSPKDVCKRKS CPNPGEIRNGQIDVPGGILFGATISFSCNTGYKLFGSTSSFCLISGSSVQWSDPLPECREIYCPAP PQIDNGIIQGERDHYGYRQSVTYACNKGFTMIGEHSIYCTVNNDEGEWSGPPPECRG |
|---|---|
| SEQ ID N-O:30 | GHCQAPDHFLFAKLKTQTNASDFPIGTSLKYECRPEYYGRPFSITCLDNLVWSSPKDVCKRKS CPNPGEIRNGQIDVPGGILFGATISFSCNTGYKLFGSTSSFCLISGSSVQWSDPLPECREIYCPAP PQIDNGIIQGERDHYGYRQSVTYACNKGFTMIGEHSIYCTVNNDEGEWSGPPPECRGKS*GHH HHHH* |
| SEQ ID N-O:31 | EDCNELPPRRNTEILTGSWSDQTYPEGTQAIYKCRPGYRSLGNVIMVCRKGEWVALNPLRKC QKRPCGHPGDTPFGTFTLTGGNVFEYGVKAVYTCNEGYQLLGEINYRECDTDGWTNDIPICE V |
| SEQ ID N-O:32 | EDCNELPPRRNTEILTGSWSDQTYPEGTQAIYKCRPGYRSLGNIIMVCRKGEWVALNPLRKC QKRPCGHPGDTPFGTFTLTGGNVFEYGVKAVYTCN GY LLGEINYRECDTDGWTNDIPICE |
| SEQ ID N-O:33 | EDCNELPPRRNTEILTGSWSDQTYPEGTQAIYKCRPGYRSLGNVIMVCRKGEWVALNPLRKC QKRPCGHPGDTPFGTFTLTGGNVFEYGVKAVYTCN GY LLGEINYRECDTDGWTNDIPICE |
| SEQ ID N-O:34 | EDCNELPPRRNTEILTGSWSDQTYPEGTQAIYKCRPGYRSLGNIIMVCRKGEWVALNPLRKC QKRPCGHPGDTPFGTFTLTGGNVFEYGVKAVYTCN GY LLGEINYRECDTDGWTNDIPICE |
| SEQ ID N-O:35 | QCNAPEWLPFARPTNLTDEFEFPIGTYLKYECRPGYYGRPFSIICLKNSVWTGAKDRCRRKSC RNPPDPVNGMVHVIKDIQFGSQIKYSCTKGYRLIGSSSATCIISGNTVIWDNETPICDRIPCGLP PTITNGDFISTNRENFHYGSVVTYRCNPGSGGRKVFELVGEPSIYCTSNDDQVGIWSGPAPQCI GHHHHHH |
| SEQ ID N-O:36 | SCEVPTRLNSASLKQPYITQNYFPVGTVVEYECRPGYRREPSLSPKLTCLQNLKWSTAVEFCK KKSCPNPGEIRNGQIDVPGGILFGATISFSCNTGYKLFGSTSSFCLISGSSVQWSDPLPECREIYC PAPPQIDNGIIQGERDHYGYRQSVTYACNKGFTMIGEHSIYCTVNNDEGEWSGPPPECRGGHH HHHH |
| SEQ ID N-O:37 | EDCNELPPRRNTEILTGSWSDQTYPEGTQAIYKCRPGYRSLGNIIMVCRKGEWVALNPLRKCQ KRPCGHPGDTPFGTFTLTGGNVFEYGVKAVYTCNEGYQLLGEINYRECDTDGWTNDIPICEV VKCLPVTAPENGKIVSSAMEPDREYHFGQAVRFVCNSGYKIEGDEEMHCSDDGFWSKEKPK CVEISCKSPDVINGSPISQKIIYKENERFQYKCNMGYEYSERGDAVCTESGWRPLPSCEEKSCD NPYIPNGDYSPLRIKHRTGDEITYQCRNGFYPATRGNTAKCTSTGWIPAPRCTLKPGHHHHHH |
| SEQ ID N-O:38 | EDCNELPPRRNTEILTGSWSDQTYPEGTQAIYKCRPGYRSLGNIIMVCRKGEWVALNPLRKCQ KRPCGHPGDTPFGTFTLTGGNVFEYGVKAVYTCNTGYKLLGEINYRECDTDGWTNDIPICEEI YCPAPPQIDNGIIQGERDHYGYRQSVTYACNKGFTMIGEHSIYCTVNNDEGEWSGPPPECRGK SGHHHHHH |

| SEQ ID NO:39 | GHCQAPDHFLFAKLKTQTNASDFPIGTSLKYECRPEYYGRPFSITCLDNLVWSSPKDVCKRKS CPNPGEIRNGQIDVPGGILFGATISFSCNTGYKLFGSTSSFCLISGSSVQWSDPLPECREIYCPAP PQIDNGIIQGERDHYGYRQSVTYACNKGFTMIGEHSIYCTVNNDEGEWSGPPPECRGGHHHH HH |
| SEQ ID NO:40 | EDCNELPPRRNTEILTGSWSDQTYPEGTQAIYKCRPGYRSLGNIIMVCRKGEWVALNPLRKCQ KRPCGHPGDTPFGTFTLTGGNVFEYGVKAVYTCNEGYQLLGEINYRECDTDGWTNDIPICEV VKCLPVTAPENGKIVSSAMEPDREYHFGQAVRFVCNSGYKIEGDEEMHCSDDGFWSKEKPK CVEISGHHHHHH |
| SEQ ID NO:41 | DCGLPPDVPNAQPALEGRTSFPEDTVITYKCEESFVKIPGEKDSVICLKGSQWSDIEEFCNRSC EVPTRLNSASLKQPYITQNYFPVGTVVEYECRPGYRREPSLSPKLTCLQNLKWSTAVEFCKKK SCPNPGEIRNGQIDVPGGILFGATISFSCNTGYKLFGSTSSFCLISGSSVQWSDPLPECREIYCPA PPQIDNGIIQGERDHYGYRQSVTYACNKGFTMIGEHSIYCTVNNDEGEWSGPPPECRGKSGH HHHHH |

## Claims

1. A hybrid protein, which comprises or consists of

    (i) CCP1-2 of human Complement Factor H (CFH),
    (ii) CCP4 of human decay accelerating factor (DAF), and

optionally a signal peptide and/or a tag.

2. The hybrid protein of claim 1, wherein CCP1 and CCP2 of CFH are directly linked together to form CCP1-2.

3. The hybrid protein of claim 1 or 2, wherein the CCP1 of human CFH comprises or consists of the amino acid sequence at positions 19-82 of the human CFH protein; and/or the CCP2 of human CFH comprises or consists of an amino acid sequence at positions 83-143 of the human CFH protein,

    wherein optionally, the CCP1 comprises a V62I mutation,
    wherein optionally, the CCP2 comprises one, two, or three of E116T, Q119K, and V143E mutations,
    wherein the amino acid positions are numbered corresponding to the amino acid positions as set forth in SEQ ID NO: 3.

4. The hybrid protein of claim 1 or 2, wherein the CCP1-2 of human CFH comprises or consists of the amino acid sequence at positions 19-143 of the human CFH protein,

    wherein optionally, the CCP1-2 comprises one, two, three, or four of V62I, E116T, Q119K, and V143E mutations,
    wherein the amino acid positions are numbered corresponding to the amino acid positions as set forth in SEQ ID NO: 3.

5. The hybrid protein of any one of claims 1-4, wherein the CCP4 of human DAF comprises or consists of the amino acid sequence at position 223-285 of the human DAF protein, wherein the amino acid positions are numbered corresponding to the amino acid positions as set forth in SEQ ID NO: 1.

6. The hybrid protein of any one of claims 1-4, wherein the CCP4 of human DAF comprises or consists of the amino acid sequence at position 223-287 of the human DAF protein, wherein the amino acid positions are numbered corresponding to the amino acid positions as set forth in SEQ ID NO: 1.

7. The hybrid protein of claim 2, wherein

(1) the CCP1 of human CFH comprises or consists of the amino acid sequences at positions 19-82 of the human CFH protein, and the CCP2 of human CFH comprises or consists of the amino acid sequence 83-143 of the human CFH protein, and the CCP4 of human DAF comprises or consists of the amino acid sequence 223-285 of the human DAF protein;

(2) the CCP1 of human CFH comprises or consists of the amino acid sequences at positions 19-82 of the human CFH protein, and the CCP2 of human CFH comprises or consists of the amino acid sequence 83-143 of the human CFH protein, and the CCP4 of human DAF comprises or consists of the amino acid sequence 223-287 of the human DAF protein;

(3) the CCP1-2 of human CFH comprises or consists of the amino acid sequence at positions 19-143 of the human CFH protein, and the CCP4 of human DAF comprises or consists of the amino acid sequence at positions 223-285 of the human DAF protein; or

(4) the CCP1-2 of human CFH comprises or consists of the amino acid sequence at positions 19-143 of the human CFH protein, and the CCP4 of human DAF comprises or consists of the amino acid sequence at positions 223-287 of the human DAF protein;

wherein the amino acid positions of the human CFH protein are numbered corresponding to the amino acid positions as set forth in SEQ ID NO: 3, and the amino acid positions of the human DAF protein are numbered corresponding to the amino acid positions as set forth in SEQ ID NO: 1.

8. The hybrid protein of claim 7, wherein the CCP1 of human CFH comprises a V62I mutation, the CCP2 of human CFH comprises one, two, or three of E116T, Q119K, and V143E mutations, and/or CCP1-2 of human CFH comprises one, two, three, or four of V62I, E116T, Q119K, and V143E mutations.

9. The hybrid protein of any one of claims 1-8, wherein the human CFH protein is a native human CFH protein or comprises

(i) the amino acid sequence as set forth in SEQ ID NO: 3 or 5;
(ii) the amino acid sequence encoded by the nucleic acid sequence as set forth in SEQ ID NO: 4 or 6;
(iii) the amino acid sequence having at least 95%, 96%, 97%, 98%, or 99% identity to the amino acid sequence as set forth in (i) or (ii); or

consists of the amino acid sequence as set forth in any one of (i) to (iii).

10. The hybrid protein of any one of claims 1-9, wherein the human DAF protein is a native human DAF protein or comprises

(i) the amino acid sequence as set forth in SEQ ID NO: 1;
(ii) the amino acid sequence encoded by the nucleic acid sequence as set forth in SEQ ID NO: 2;
(iii) the amino acid sequence having at least 95%, 96%, 97%, 98%, or 99% identity to the amino acid sequence as set forth in (i) or (ii); or

consists of the amino acid sequence as set forth in any one of (i) to (iii).

11. The hybrid protein of any one of claims 1-10, wherein
the CCP1 of human CFH comprises or consists of the amino acid sequence as set forth in SEQ ID NO: 14 or 15, or an amino acid sequence having at least 95%, 96%, 97%, 98%, or 99% identity to the amino acid sequence as set forth in SEQ ID NO: 14 or 15;
the CCP2 of human CFH comprises or consists of the amino acid sequence as set forth in SEQ ID NO: 16 or 17, or an amino acid sequence having at least 95%, 96%, 97%, 98%, or 99% identity to the amino acid sequence as set forth in SEQ ID NO: 16 or SEQ ID NO: 17;

the CCP1-2 of human CFH comprises or consists of the amino acid sequence as set forth in SEQ ID NO: 31, 32, 33, or 34, or an amino acid sequence having at least 95%, 96%, 97%, 98%, or 99% identity to the amino acid sequence as set forth in SEQ ID NO: 31, 32, 33, or 34; and/or
the CCP4 of human DAF comprises or consists of the amino acid sequence as set forth in SEQ ID NO: 10 or SEQ ID NO: 11, or an amino acid sequence having at least 95%, 96%, 97%, 98%, or 99% identity to the amino acid sequence as set forth in SEQ ID NO: 10 or SEQ ID NO: 11.

12. The hybrid protein of any one of claims 1-11, wherein the tag is, for example, a purification tag such as a hexahistidine tag or a biotin tag, comprising, e.g., the amino acid sequence as set forth in SEQ ID NO: 19.

13. The hybrid protein of any one of claims 1-12, wherein the signal peptide is secretory, comprising, e.g., the amino acid sequence as set forth in SEQ ID NO: 20.

14. The hybrid protein of any one of claims 1-13, wherein the hybrid protein comprises or consists of an amino acid sequence of any one of SEQ ID NOs: 21-28, or an amino acid sequence having at least 95%, 96%, 97%, 98%, or 99% identity to the amino acid sequence.

15. A hybrid protein, which comprises or consists of

    (i) CCP15 or CCP8 of human complement receptor 1 (CR1),
    (ii) CCP3-4 of human decay accelerator factor (DAF), and

    optionally a signal peptide and/or a tag.

16. The hybrid protein of claim 15, wherein the CCP3 and CCP4 of DAF are directly linked together to form CCP3-4.

17. The hybrid protein of claim 15 or 16, wherein CCP15 of human CR1 comprises the amino acid sequence at positions 941-1001 of the human CFH protein, and CCP8 comprises the amino acid sequence at positions 491-551 of the human CR1 protein, wherein the amino acid positions are numbered corresponding to the amino acid positions as set forth in SEQ ID NO: 3.

18. The hybrid protein of any one of claims 15-17, wherein the CCP3 of human DAF comprises or consists of the amino acid sequence at positions 161-222 of the human DAF protein, and/or the CCP4 of human DAF comprises or consists of the amino acid sequence at positions 223-285 of the human DAF protein, wherein the amino acid positions are numbered corresponding to the amino acid positions as set forth in SEQ ID NO: 1.

19. The hybrid protein of any one of claims 15-17, wherein the CCP3 of human DAF comprises or consists of the amino acid sequence at positions 161-222 of the human DAF protein, and/or the CCP4 of human DAF comprises or consists of the amino acid sequence at positions 223-287 of the human DAF protein, wherein the amino acid positions are numbered corresponding to the amino acid positions as set forth in SEQ ID NO: 1.

20. The hybrid protein of claim 16 or 17, wherein the CCP3-4 of human DAF comprises or consists of the amino acid sequence at positions 161-285 of the human DAF protein, wherein the amino acid positions are numbered corresponding to the amino acid positions as set forth in SEQ ID NO: 1.

21. The hybrid protein of claim 16 or 17, wherein the CCP3-4 of human DAF comprises or consists of the amino acid sequence at positions 161-287 of the human DAF protein, wherein the amino acid positions are numbered corresponding to the amino acid positions as set forth in SEQ ID NO: 1.

22. The hybrid protein of claim 16, wherein

    (1) the CCP15 of human CR1 comprises or consists of the amino acid sequence at positions 941-1001 of the human CR1 protein, and the CCP3 of human DAF comprises or consists of the amino acid sequence at positions 161-222 of the human DAF protein, and the CCP4 of human DAF comprises or consists of the amino acid sequence at positions 223-285 of the human DAF protein; or
    (2) the CCP15 of human CR1 comprises or consists of the amino acid sequence at positions 941-1001 of the human CR1 protein, and the CCP3 of human DAF comprises or consists of the amino acid sequence at positions 161-222 of the human DAF protein, and the CCP4 of human DAF comprises or consists of the amino acid sequence at positions 223-287 of the human DAF protein; or
    (3) the CCP15 of human CR1 comprises or consists of the amino acid sequence at positions 941-1001 of the human CR1 protein, and the CCP3-4 of human DAF comprises or consists of the amino acid sequence at positions 161-285 of the human DAF protein;
    (4) the CCP15 of human CR1 comprises or consists of the amino acid sequence at positions 941-1001 of the human CR1 protein, and the CCP3-4 of human DAF comprises or consists of the amino acid sequence at positions 161-287 of the human DAF protein;

wherein the amino acid positions of the human CR1 protein are numbered corresponding to the amino acid positions as set forth in SEQ ID NO:7, and the amino acid positions of the human DAF protein are numbered corresponding to the amino acid positions as set forth in SEQ ID NO: 1.

23. The hybrid protein of any one of claims 16-22, wherein the human CR1 protein is a native human CR1 protein or comprises

(i) the amino acid sequence as set forth in SEQ ID NO: 7;
(ii) the amino acid sequence encoded by the nucleic acid sequence as set forth in SEQ ID NO: 8;
(iii) the amino acid sequence having at least 95%, 96%, 97%, 98%, or 99% identity to the amino acid sequence as set forth in (i) or (ii); or

consists of the amino acid sequence as set forth in any one of (i) to (iii).

24. The hybrid protein of any one of claims 15-23, wherein the human DAF protein is a native human DAF protein or comprises

(i) the amino acid sequence as set forth in SEQ ID NO: 1;
(ii) the amino acid sequence encoded by the nucleic acid sequence as set forth in SEQ ID NO: 2;
(iii) the amino acid sequence having at least 95%, 96%, 97%, 98%, or 99% identity to the amino acid sequence as set forth in (i) or (ii); or

consists of the amino acid sequence as set forth in any one of (i) to (iii).

25. The hybrid protein of any one of claims 18-24, wherein

the CCP15 or CCP8 of human CR1 comprises or consists of the amino acid sequence as set forth in SEQ ID NO: 18, or an amino acid sequence having at least 95%, 96%, 97%, 98%, or 99% identity to the amino acid sequence as set forth in SEQ ID NO: 18;
the CCP3 of human DAF comprises or consists of the amino acid sequence as set forth in SEQ ID NO: 9, or an amino acid sequence having at least 95%, 96%, 97%, 98%, or 99% identity to the amino acid sequence as set forth in SEQ ID NO: 9;
the CCP4 of human DAF comprises or consists of the amino acid sequence as set forth in SEQ ID NO: 10 or SEQ ID NO: 11, or an amino acid sequence having at least 95%, 96%, 97%, 98%, or 99% identity to the amino acid sequence as set forth in SEQ ID NO: 10 or SEQ ID NO: 11;
the CCP3-4 of human DAF comprises or consists of the amino acid sequence as set forth in SEQ ID NO: 12 or SEQ ID NO: 13, or an amino acid sequence having at least 95%, 96%, 97%, 98%, or 99% identity to the amino acid sequence as set forth in SEQ ID NO: 12 or SEQ ID NO: 13.

26. The hybrid protein of any one of claims 16-25, wherein the tag is, for example, a purification tag such as a hexahistidine tag or a biotin tag, comprising, e.g., the amino acid sequence as set forth in SEQ ID NO: 19.

27. The hybrid protein of any one of claims 16-26, wherein the signal peptide is secretory, comprising, e.g., the amino acid sequence as set forth in SEQ ID NO: 20.

28. The hybrid protein of any one of claims 16-27, wherein the hybrid protein comprises or consists of an amino acid sequence as set forth in SEQ ID NO: 29 or 30, or an amino acid sequence having at least 95%, 96%, 97%, 98%, or 99% identity to the amino acid sequence.

29. A nucleic acid molecule encoding the hybrid protein of any one of claims 1-28.

30. An expression vector comprising the nucleic acid molecule of claim 29, preferably the expression vector is pcDNA3.1.

31. A host cell comprising the nucleic acid molecule of claim 29 or the expression vector of claim 30, preferably the host cell is prokaryotic or eukaryotic, such as a 293 cell, e.g., an Expi293 cell.

32. A method of preparing the hybrid protein of any one of claims 1-28, the method comprising culturing the host cell of claim 31 under conditions suitable for expression of the hybrid protein, and optionally further comprising isolating the protein from the host cell or the host cell culture medium, and/or purifying the protein.

33. A pharmaceutical composition or medicament or formulation comprising the hybrid protein according to any one of claims 1-28 or a nucleic acid molecule encoding the same, or the nucleic acid molecule of claim 29, and optionally a pharmaceutically acceptable excipient.

34. A pharmaceutical combination product comprising
the hybrid protein according to any one of claims 1-28 or a nucleic acid molecule encoding the same, or the nucleic acid molecule of claim 29; and additional therapeutic agents.

35. A method of preventing or treating a complement system-associated disease or disorder in a subject, comprising administering to the subject an effective amount of the hybrid protein of any one of claims 1-28 or a nucleic acid molecule encoding the same; or the nucleic acid molecule of claim 29; or the pharmaceutical composition or formulation of claim 33; or the pharmaceutical combination product of claim 34.

36. The method of claim 35, wherein the disease or disorder is due to an abnormal activation of the complement system or a dysregulation of the complement system.

37. The method of claim 36, wherein the abnormal activation of the complement system or the dysregulation of the complement system is due to, for example, a microbial infection or an increase in autoimmune antibodies, or a decrease, deletion, incapacitation, or interference or blockade of a function of a complement regulatory protein.

38. The method of claim 37, wherein the complement system-associated disease or disorder is selected from a disease caused by activation of the alternative pathway, such as a disease requiring inhibition of the alternative pathway of the complement immunity and optionally not inhibition of the classical pathway of the complement immunity, such as paroxysmal nocturnal hemoglobinuria (PNH).

CFH CCP1-4
PDB: 5o35

CFH CCP1-2

F12D4 (杂合蛋白)

CD97
EGF-like domain 1-3

DAF CCP4

DAF CCP1-4
PDB: 7do4

Fig. 1

| | M | Marker |
|---|---|---|
| | 1 | CR13m |
| | 2 | FH15 |
| | 3 | D24 |
| | 4 | CR15D34 |
| | 5 | FH13 |
| | 6 | F12D4 |
| | 7 | D14 |

Fig. 2

| RT [min] | Name | Area | Main peak% |
|---|---|---|---|
| 13.966 | | 58.7 | 1.665 |
| 15.308 | | 3464.4 | 98.276 |
| 17.169 | | 2.1 | 0.059 |
| | Sum | 3525.2 | |

Fig. 3

| RT [min] | Name | Area | Main peak% |
|---|---|---|---|
| 8.480 | | 1147.1 | 26.863 |
| 15.964 | | 3007.3 | 70.427 |
| 17.245 | | 115.7 | 2.711 |
| | Sum | 4270.1 | |

Fig. 4

## CP assay

| | CR13m | FH15 | D24 | CR15D34 | FH13 | F12D4 | D14 |
|---|---|---|---|---|---|---|---|
| IC50 | 4.716 | 424.6 | 6.231 | ~ 7837 | ~ 2.706e-025 | 530.4 | 92.52 |

Fig. 5

## AP assay

| | CR13m | FH15 | F12D4 |
|---|---|---|---|
| IC50 | 172.9 | 281.4 | 55.36 |

## AP assay

| | D24 | CR15D34 | FH13 | F12D4 | D14 |
|---|---|---|---|---|---|
| IC50 | 220.1 | 120.6 | ~3909525310 | 31.97 | 1646 |

Fig. 6

## INTERNATIONAL SEARCH REPORT

| International application No. |
| --- |
| **PCT/CN2023/094269** |

| A. | CLASSIFICATION OF SUBJECT MATTER |
| --- | --- |

C07K19/00(2006.01)i; C07K14/47(2006.01)i; C12N15/62(2006.01)i; A61K38/17(2006.01)i

According to International Patent Classification (IPC) or to both national classification and IPC

| B. | FIELDS SEARCHED |
| --- | --- |

Minimum documentation searched (classification system followed by classification symbols)

    IPC: C07K; C12N; A61K

Documentation searched other than minimum documentation to the extent that such documents are included in the fields searched

Electronic data base consulted during the international search (name of data base and, where practicable, search terms used)

    DWPI, VEN, CNABS, CNTXT, USTXT, EPTXT, WOTXT, 万方数据库, WANFANG DATABASE, CNKI, PubMed, ISI web of Knowledge, GenBank, EBI, STN: 补体因子, 衰变加速因子, 衰变加快因子, 促衰变因子, 融合, 杂合, 重复模块, 重复片段, 补体控制蛋白, 补体途径, CCP, DAF, CFH, FH, SCR, decay accelerating factor, CD55, complement factor H portein, H 因子, complement component, complement control protein repeats, SEQ ID NOs: 1-33, 申请人, applicant, 发明人, inventor, CR1, 补体受体1, CD35

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 109689084 A (UNIVERSITAT ULM) 26 April 2019 (2019-04-26)<br>  claims 1-24 | 15-38 |
| Y | CN 109689084 A (UNIVERSITAT ULM) 26 April 2019 (2019-04-26)<br>  claims 1-24 | 1-14, 29-38 |
| Y | CN 113365648 A (UNIVERSITAT ULM) 07 September 2021 (2021-09-07)<br>  claims 1-17 | 1-14, 29-38 |
| X | WO 2005069726 A2 (CASE WESTERN RESERVE UNIVERSITY) 04 August 2005 (2005-08-04)<br>  claims 1-15 | 15-38 |
| A | CN 106928371 A (QUASSIA BIOPHARMA CO., LTD.) 07 July 2017 (2017-07-07)<br>  entire document | 1-38 |

☑ Further documents are listed in the continuation of Box C.      ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **06 December 2023** | **12 December 2023** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/094269** |

### C. DOCUMENTS CONSIDERED TO BE RELEVANT

| Category* | Citation of document, with indication, where appropriate, of the relevant passages | Relevant to claim No. |
| --- | --- | --- |
| A | WO 2018197873 A1 (UNIVERSITY OF NEWCASTLE UPON TYNE) 01 November 2018 (2018-11-01)<br>  entire document | 1-38 |
| A | 冯帆等 (FENG, Fan et al.). "含有补体控制蛋白结构域的蛋白质的结构和功能 (Structure and Function of Complement Control Protein Module-Containing Proteins)"<br>*生物技术通讯 (Letters in Biotechnology)*, Vol. 21, No. (2), 31 March 2010 (2010-03-31), pp. 258-261 | 1-38 |
| A | JÖNSEN, A. et al. "Mutations in Genes Encoding Complement Inhibitors CD46 and CFH Affect the Age at Nephritis Onset in Patients with Systemic Lupus Erythematosus"<br>*Arthritis Research & Therapy*, 15 December 2011 (2011-12-15),<br>  R206, pages 1-9 | 1-38 |
| A | SAGE, M.A.G. et al. "A Factor H-Fc Fusion Protein Increases Complement-mediated Opsonophagocytosis and Killing of Community Associated Methicillin-resistant Staphylococcus aureus"<br>*Plos One*, Vol. 17, No. (3), 24 March 2022 (2022-03-24),<br>  Article e0265774 | 1-38 |

Form PCT/ISA/210 (second sheet) (July 2022)

**INTERNATIONAL SEARCH REPORT**

International application No.

**PCT/CN2023/094269**

| Box No. I | Nucleotide and/or amino acid sequence(s) (Continuation of item 1.c of the first sheet) |

1.  With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT

| International application No. |
| --- |
| **PCT/CN2023/094269** |

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **35-38**
because they relate to subject matter not required to be searched by this Authority, namely:

Claims 35-38 relate to a method for preventing or treating a complement system-related disease or condition in a subject, that is, claims 35-38 relate to subject matter for which no search is required by the International Searching Authority as defined in PCT Rule 39.1: (4) methods for treatment of the human or animal body by surgery or therapy, as well as diagnostic methods. The international search is carried out on the basis of the use in the preparation of a drug for prevention or treatment.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2023/094269**

| Patent document cited in search report | | | Publication date (day/month/year) | Patent family member(s) | | | Publication date (day/month/year) |
|---|---|---|---|---|---|---|---|
| CN | 109689084 | A | 26 April 2019 | WO | 2018002131 | A1 | 04 January 2018 |
| | | | | AU | 2017289214 | A1 | 24 January 2019 |
| | | | | AU | 2017289214 | B2 | 18 November 2021 |
| | | | | CA | 3029106 | A1 | 04 January 2018 |
| | | | | MX | 2019000059 | A | 07 September 2020 |
| | | | | US | 2019202878 | A1 | 04 July 2019 |
| | | | | PL | 3474883 | T3 | 19 September 2022 |
| | | | | IL | 263949 | A | 31 January 2019 |
| | | | | JP | 2019523238 | A | 22 August 2019 |
| | | | | JP | 7068203 | B2 | 16 May 2022 |
| | | | | ES | 2925466 | T3 | 18 October 2022 |
| | | | | EP | 3474883 | A1 | 01 May 2019 |
| | | | | EP | 3474883 | B1 | 25 May 2022 |
| | | | | KR | 20190039090 | A | 10 April 2019 |
| | | | | KR | 102566634 | B1 | 14 August 2023 |
| | | | | DK | 3474883 | T3 | 29 August 2022 |
| CN | 113365648 | A | 07 September 2021 | US | 2021355178 | A1 | 18 November 2021 |
| | | | | WO | 2019243586 | A1 | 26 December 2019 |
| | | | | EP | 3810166 | A1 | 28 April 2021 |
| | | | | EP | 3586860 | A1 | 01 January 2020 |
| | | | | JP | 2021527439 | A | 14 October 2021 |
| WO | 2005069726 | A2 | 04 August 2005 | US | 2008188404 | A1 | 07 August 2008 |
| | | | | US | 8124097 | B2 | 28 February 2012 |
| | | | | US | 2012226020 | A1 | 06 September 2012 |
| | | | | US | 8932601 | B2 | 13 January 2015 |
| | | | | WO | 2005069726 | A3 | 18 January 2007 |
| CN | 106928371 | A | 07 July 2017 | WO | 2017114401 | A1 | 06 July 2017 |
| | | | | US | 2019071477 | A1 | 07 March 2019 |
| WO | 2018197873 | A1 | 01 November 2018 | GB | 201707248 | D0 | 21 June 2017 |
| | | | | US | 2023101835 | A1 | 30 March 2023 |
| | | | | GB | 201706808 | D0 | 14 June 2017 |
| | | | | EP | 3615559 | A1 | 04 March 2020 |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9988611 B2

**Non-patent literature cited in the description**

- The Complement FactsBook. Elsevier Ltd., 2018
- **R.C.ROWE** ; **P.J. SESKEY** ; **S.C. OWEN**. Handbook of Pharmaceutical Excipients. Pharmaceutical Press
- **HUI-FEN ZHANG et al.** *THE JOURNAL OF BIOLOGICAL CHEMISTRY*, 20 July 2001, vol. 276 (29), 27290-27295
- **MASHA FRIDKIS-HARELI et al.** *Blood.*, 27 October 2011, vol. 118 (17), 4705-4713
- **AGUSTÍN TORTAJADA et al.** *Hum Mol Genet.*, 15 September 2009, vol. 18 (18), 3452-3461
- **JÄGER, V. et al.** *BMC Biotechnol*, 2013, vol. 13, 52